(19) Europäisches Patentamt
European Patent Office
Office européen des brevets

(11) EP 1 487 791 B1

(12) **EUROPEAN PATENT SPECIFICATION**

(45) Date of publication and mention of the grant of the patent:
**02.07.2008 Bulletin 2008/27**

(21) Application number: **02718466.2**

(22) Date of filing: **25.03.2002**

(51) Int Cl.:
***C07D 207/20*** *(2006.01)* ***A61K 31/10*** *(2006.01)*
***A61P 31/10*** *(2006.01)*

(86) International application number:
**PCT/IB2002/001207**

(87) International publication number:
**WO 2003/080572 (02.10.2003 Gazette 2003/40)**

(54) **A NOVEL ANTIFUNGAL MOLECULE 2-(3,4-DIMETHYL-2,5-DIHYDRO-1H-PYRROL-2-YL)-METHYLETHYL PENTANOATE**

EINE NEUE ANTIFUNGALE VERBINDUNG 2-(3,4-DIMETHYL-2,5-DIHYDRO-1H-PYRROL-2-YL)-METHYLETHYL PENTANOATE

NOUVELLE MOLECULE ANTIFONGIQUE DE 2-(3,4-DIMETHYL-2,5-DIHYDRO-1H-PYRROL-2-YL)-METHYLETHYL PENTANOATE

(84) Designated Contracting States:
**AT BE CH CY DE DK ES FI FR GB GR IE IT LI LU MC NL PT SE TR**

(43) Date of publication of application:
**22.12.2004 Bulletin 2004/52**

(73) Proprietor: **COUNCIL OF SCIENTIFIC AND INDUSTRIAL RESEARCH**
**New Delhi 100 001 (IN)**

(72) Inventors:
- **SHARMA, Ganda, Lal**
  **Centre for Biochem.Technology**
  **110 007 Delhi (IN)**
- **RAJESH, R.**
  **Centre for Biochemical Technology**
  **110 007 Delhi (IN)**
- **ALI, Mohammad**
  **Hamdard College of Pharmacy**
  **110 062 New Delhi (IN)**

(74) Representative: **Moy, David et al**
**Appleyard Lees**
**15 Clare Road**
**Halifax HX1 2HY (GB)**

(56) References cited:
- **CHEMICAL ABSTRACTS, vol. 125, no. 21, 18 November 1996 (1996-11-18) Columbus, Ohio, US; abstract no. 268036, ZHANG, SHUMING ET AL: "Studies on the dispelling dynamics of metalaxyl in Hindu datura ( Datura metel )" XP002204182 & ZHONGCAOYAO (1996), 27(7), 403-405 ,**
- **SINGH, U. P. ET AL: "Antifungal activity of withametelin, a withanolide isolated from Datura metel" MYCOBIOLOGY (2001), 29(2), 96-99 , XP001084734**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 487 791 B1

## Description

### Technical Field

The present invention relates to a novel antifungal molecule 2-(3,4-dimethyl-2,5-dihydro-1H-pyrrol-2-yl)-1-methylethyl pentanoate of formula (I). It particularly relates to a novel antifungal lead molecule isolated from a plant Datura *metel.*

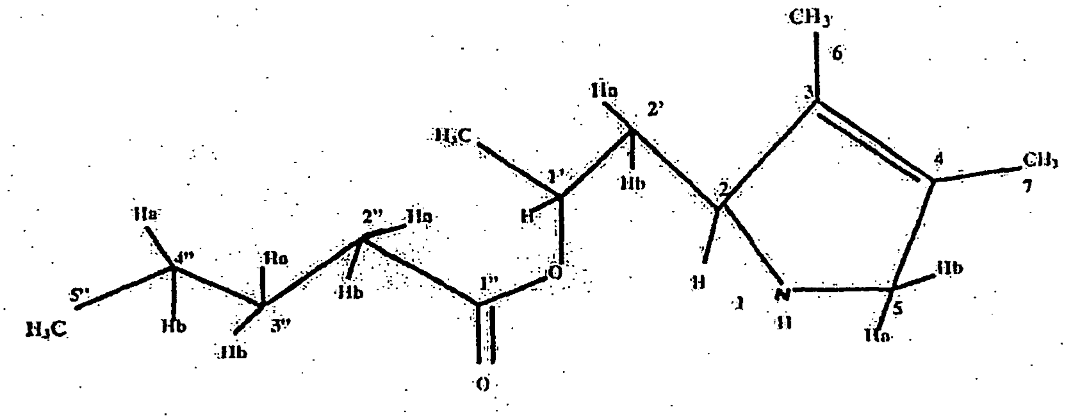

**Formula (1)**

The main utility of the invention is to provide a new lead molecule for development of new drugs for treating diseases caused by pathogenic fungi.

### Background Art

The incidence and mortality due to mycotic infections has been observed to be on the rise in the past decade (Saral, R., 1991, Rev. Inf. Dis. 13, 487-492., Koll, B.S. and Brown, A.E. 1993, Clin. Inf. Dis. 17, S-322-S-326). Infections induced by these species are increasingly recognized as emerging threat to the public health. In immuno-compromised host, the infections by *Aspergillus, Candida, Histoplasma* etc. become invasive and disseminate from primary site of infection to other parts of the body including gut, kidney, brain etc. Invasive aspergillosis is reported to be associated with a mortality rate of 55% (Denning, D.W. and Stevens, D.A., 1990, Rev. Inf. Dis., 12, 1147-1201). Mortality due to *Aspergillus* infection in bone marrow transplant recipients was observed to be as high as 80% despite appropriate chemotherapy (Meyer, J.D., 1990, Semin. Oncol. 17 (suppl. 6), 10-13). Cerebral aspergillosis presents the symptoms of acute meningitis and is always fatal. *Candida* species are commensal organisms of human mucocutaneous surface. However, in immunocompromised host, *Candida* may progress to become opportunistic potential pathogen. It may be transmitted by contact to other immuno-compromised patients as a nosocomial pathogen.

It is, therefore, important to diagnose and treat these fungal infections at early stage to prevent irreversible damage. For the treatment of mycoses, several compounds belonging to azoles, polyenes and other groups of chemicals have been described in the literature. But all of these compounds have their own limitations.

Polyenes are among the oldest and most frequently prescribed antifungal agents. Amphotericin B is an important polyene drug which has been found to have a broad range activity against fungi, but its therapeutic doses are frequently associated with severe chills, fever, vomiting, life threatening hypotension and respiratory distress. Amphotericin B produces renal dysfunction (Allende, M.C., Lee, J.W., Francis, P., Garrett, K., Dollenberg, H., Berenguer, J., Lyman, C. A., Pizzo, P.A. and Walsh, T.J. 1994, Antimicrob. Agents and Chemother. 38, 518-522) and necessity of its intravenous administration is its major limitation (Stevens, D.A. and Lee, J.Y., 1997, Arch. Int. Med., 57, 1857-1862). In addition to its adverse effects, Amphotericin B resistance in fungal species has been reported (Gokhale, P.C., Kshirsagar, N.A. and Pandya, S.K., 1993, Current Science 65(6), 448-454, Forthergill, A.W. and McGough, D.A., 1995, Clin. Microbiol. Procedure Handbook, In Vitro susceptibility Testing of Yeast, 5.15.1-5.15.15).

Nystatin is another important drug, however, the development of resistance and its dose limited toxicity make this drug also less useful (Forthergill A.W. and McGough A.W., 1995, Clin. Microbiol. Procedure Handbook, In Vitro susceptibility Testing of Yeasts, 5.15.1-5.15.15).

New azole antifungals such as ketoconazole, fluconazole and itraconazole provided the hope that these compounds might be useful to prevent or treat fungal infections. However, their liver toxicity, hypoglycemic nature and development of resistant strains of fungi did not keep this hope alive for longer period (Denning, D.W. Tucker, R.M., Hanson, L.H., Hamilton, J.R. and Stevens, D.A., 1989, Arch. Int. Med., 149, 2301-2308). The azoles are reported to decrease the secretion of stomach acid and thereby reducing the absorption of drug itself and other important biomolecules. These effects may cause severe drop in body glucose to a level which may be life threatening. Resistance against the azole derivatives also has been reported in fungi (Forthergill A.W. and McGough A.W., 1995, Clin. Microbiol. Procedure Handbook, In Vitro susceptibility Testing of Yeasts, 5.15.1-5.15.14).

Currently available antifungal drugs are not sufficiently broad in their spectrum and not consistently effective against fungi including Aspergilli. These drugs are either toxic or immunosuppressive in nature. Further, the development of resistance in fungi to most of available drugs has also been reported.

It is evident from the above that need exists for development of new antifungal drugs which obviate the drawbacks listed above. The novelty of the present invention is to provide a process for isolation and characterization of a novel antifungal lead molecule from *Datura metel,* which is many fold less cytotoxic as compared to standard antifungal drug such as amphotericin B.

### Object of the Invention

The main object of the present invention is to provide a novel antifungal lead compound 2-(3,4-dimethyl-2,5-dihydro-1H-pyrrol-2-yl)-1'-methylethyl pentanoate for developing new drugs against the pathogenic fungi.

Another object of the invention is to provide the process for isolation of the novel antifungal lead molecule 2-(3,4-dimethyl-2,5-dihydro-1H-pyrrol-2-yl)-1'-methylethyl pentanoate from a plant *Datura metel.*

Still another object of the present invention is to provide a method for testing the novel compound 2-(3,4-dimethyl-2,5-dihydro-1H-pyrrol-2-yl)-1'-methylethyl pentanoate as an antifungal agent.

Yet another object of the invention is to provide usage of the novel compound 2-(3,4-dimethyl-2,5-dihydro-1H-pyrrol-2-yl)-1'-methylethyl pentanoate as an antifungal agent.

Still yet another object is to provide a novel antifungal lead molecule 2-(3,4-dimethyl-2,5-dihydro-1H-pyrrol-2-yl)-1'-methylethyl pentanoate which is 57.8 times less cytotoxic than the standard antifungal drug such as Amphotericin B.

### Summary of the invention

The present invention is directed to a novel antifungal compound 2-(3,4 dimethyl-2,5-dihydro-1H-pyrrole-2-yl)-1'-methylethyl pentanoate of the formula (1) as given below.

The compound has antifungal properties and is several times less cytotoxic as compared to the standard antifungal drugs. This compound may be useful for controlling systemic and superficial fungal infections in humans with fewer toxic effects. This compound has been isolated from an easily available plant *Datura metel*.

### Brief description of accompanying drawings

Fig 1: Photograph of a plate showing TLC pattern of the active column sub-fractions and purified compound.
Fig 2: HPLC profile of TLC purified compound
Fig 3: Photograph of TLC of HPLC purified compound.
Fig 4: HPLC profile of purified compound.
Fig 5: Inhibition of growth of *A. fumigatus* by purified compound.
Fig 6: Graph represents the percent spore germination inhibition by the compound against *Aspergillus* species.
Fig 7: UV spectrum of the compound.
Fig 8: IR spectrum of the compound.
Fig 9: Mass spectrum of the compound
Fig 10: NMR spectrum of the compound.
Fig 11: COSY spectrum of the compound.
Fig 12: Percent cytotoxicity of the compound
Fig 13: The dose required to protect 50% of the animals ($ED_{50}$) after treatment with PC-1.

### Detailed Description of the Invention

Accordingly the present invention provides a novel antifungal molecule 2-(3,4-dimethyl-2,5-dihydro-1H-pyrrol-2-yl)-1'-methylethyl pentanoate of formula 1

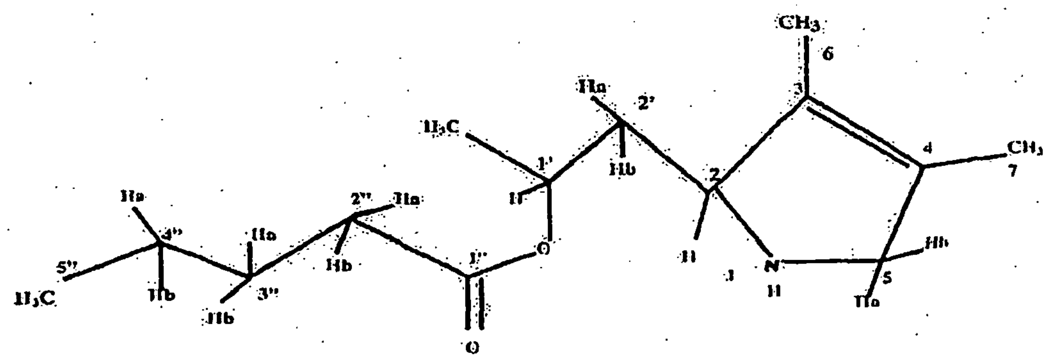

**Formula 1**

In an embodiment, the characterization of the active antifungal lead molecule is carried out by known analytical methods such as TLC, group specific chemical staining, UV, infra-red, mass and nuclear magnetic resonance spectroscopy and biological assays such as antimycotic assay and cytotoxicity assay.

In still another embodiment of the invention, the novel antifungal lead molecule 2-(3,4-dimethyl-2,5-dihydro-1H-pyrrol-2-yl)-1-methylethyl pentanoate is a heterocylic alkaloid as shown by group specific chemical staining using Dragendorff reagent and has following characteristics:

$R_f$ value of 0.22 on TLC (Fig 3),

Absorption maixma at 300 nm and 206 nm in UV spectrum (Fig. 7)

IR $V_{Max:}$ 3434 (NH), 3024, 1711 (ester), 1646 (C=C), 1524, 1426,1230, 938, 760 $cm^{-1}$ (Fig 8).

+ve FABMS m/z: 239 $[M]^+$ ($C_{14} H_{25} O_2$ N) (16.3), 212 (13.2), 174 (16.2), 122 (34.2), 102 (96.4), 58 (100), and 57 (19.2) (Fig 9).

$^1H$ NMR ($CDCl_3$): $\delta$ 4.15 (1H, m, $C_{1'}$-H), 3.75 (1H, m, $C_2$-H) 3.17 (2H, dd, J=7.44 Hz, 7.44 Hz, $C_5$ - $H_a$ and $C_5$ - $H_a$ and $C_5$-$H_b$) 2.31 (1 H, t, J=6.48 Hz, $C_{2''}$ -$H_a$), 1.95 (1 H, m, $C_2$" -$H_b$) 1.62 (6H, br, $C_6$-$CH_3$ and $C_7$-$CH_3$), 1.42 (1H, d, J = 6.96 Hz, $C_{2'}$-$H_a$), 1.35 (1H, d, J=7.28 Hz, $C_{2'}$-$H_b$), 1.25 (7H, br, $C_{1'}$-$CH_3$, $C_{3''}$-$H_a$, $C_{3''}$-$H_b$, $C_{4''}$-$H_a$ and $C_{4''}$-$H_b$), 0.87 (3H, t, J = 5.96 Hz, $C_{5''}$-$CH_3$) and COSY spectrum (Fig 11).

The minimum inhibitory concentration (MIC) of the said novel antifungal lead molecule is 5.0 μg/disc by disc diffusion assay (Fig. 5) and 87.5 μg/ml by percent spore germination inhibition (Fig. 6) respectively.
The dose cytotoxic to 50% of the cells ($CT_{50}$ value) of the said antifungal molecule is 889.2 μg/ml (Fig 12).
The $ED_{50}$ for the said antifungal compound is 167.0-mg/kg body weight (Fig 13).
The antifungal effective dose to the said subject ranges from 100 to 400 mg/kg body weight (Table 3).

In another embodiment, the novel anti-fungal compound is 57.8 times less cyto-toxic than the standard anti-fungal drug.

In further embodiment is provided a method for treatment as wherein, the dose of 2-(3,4-dimethyl-2,5-dihydro-1H-pyrrol-2-yl)-1-methylethyl pentanoate, effective for survival of 50% ($ED_{50}$) of the said subjects is 167.0 mg/kg body weight (Fig. 13).

In still another embodiment, the protective *in vivo* effective dose of the antifungal compound 2-(3,4-dimethyl-2,5-dihydro-1H-pyrrol-2-yl)-1'-methylethyl pentanoate to the said subject ranges from 100 to 400 mg/kg body weight (Table 3).

Yet another embodiment of the invention provides a pharmaceutical composition comprising an acceptable amount of above compound 2-(3,4-dimethyl-2,5-dihydro-1H-pyrrol-2-yl)-1'-methylethyl pentanoate with pharmaceutically acceptable additives and adjuvants.

Yet another embodiment, the acceptable additives are selected from the group of nutrients, which are pharmaceutically acceptable carrier.

Yet another embodiment, the novel antifungal molecule may be used in the form of tablet, capsule, syrup, powder, gel, ointment and injection.

Yet, another embodiment is to provide the pharmaceutically acceptable composition contains the effective amount of novel molecule at a concentration in the range of 100 to 400mg/ml.

In still yet another embodiment, is to provide a method for the treatment of fungal infections in a said subject comprising the steps of administration of an effective amount of 2-(3,4 dimethyl-2,5-dihydro-1h-pyrrole-2-yl)-1'-methylethyl pentanoate through routes such as oral, nasal, intra venous, intra-peritoneal, intra muscular etc.

In an embodiment, the effective dose of 2-(3,4-dimethyl-2, 5-dihydro-1H-pyrrol-2-yl)-1'-methylethyl pentanoate may be in the range of 100-400 mg/kg of body weight.

One more embodiment of the invention relates to a process for isolation of a novel antifungal compound 2-(3,4 dimethyl-2,5-dihydro-1H-pyrrole-2-yl)-1'-methylethyl pentanoate, from a plant, *Datura metel ,* which comprises (i) extracting successively the powdered *Datura metel* plant material with an organic solvent at a temperature range of 15-45°C, (ii) removing the solvent to obtain residue, (iii) extracting the residue of step (ii) with an aliphatic hydrocarbon solvent followed by extraction with chloroform, (iv) removing chloroform from chloroform fraction of step (iii), (v) screening the residue of step (iv) obtained from chloroform fraction for antimycotic activity, (vi) isolating and purifying the novel antifungal lead molecule from active antimicotic chloroform fraction by adopting conventional chromatographic methods, and (vii) assaying the said pure lead molecule for antifungal activity and its cytotoxicity.

One more embodiment of the present invention, wherein the solvent used for extraction is selected from the group consisting of alcoholic solvent, ketonic solvent and/or halogenated hydrocarbon.

Still another embodiment of the invention, the solvent used for extraction is preferably selected from group consisting of methanol, ethanol, acetone and chloroform.

Still another embodiment of the invention, wherein the aliphatic hydrocarbon solvent used is selected from hexane, petroleum ether.

In yet another embodiment, wherein the compound is purified by using column or thin layer chromatography (TLC) and high performance liquid chromatography (HPLC).

In yet another embodiment to the invention, the purification of novel compound may be carried out by Thin Layer Chromatography using solvent systems selected from Chloroform: Acetone: Diethylamine (5.0:4.0:1.0), Chloroform: Methanol: Diethylamine (8.5:1.5:0.1) and Chloroform: Methanol: Formic acid (8.0:2.0:0.1) or different combinations of above said organic solvents.

In yet another embodiment of the invention, the purification of novel compound may be effected by HPLC using solvent system 70:30 of acetonitrile and water using reverse phase RP-8 column.

In still yet another embodiment of the invention, the characterization of active antifungal lead molecule is carried out by known analytical methods such as, using TLC, group staining reagent, infra red, ultra violet, mass and nuclear magnetic resonance spectroscopy and biological assays such as antimycotic and cytotoxic assay.

In still another embodiment, the invention provides a method of testing of novel compound (2-(3,4-dimethyl-2,5-dihydro-1H-pyrrol-2-yl)-1'-methylethyl pentanoate) as an antifungal agent.

In still yet another embodiment to the present invention, the antimycotic activity may be tested after each purification step by known methods such as microbroth dilution (MD), disc diffusion (DD) and spore germination inhibition (SGI).

**Purification of the compound**

The invention provides a novel antifungal molecule 2-(3,4-dimethyl-2,5-dihydro-1H-pyrrol-2-yl)-1'-methylethyl pentanoate a process for its isolation. It particularly relates to the isolation of a novel antifungal lead molecule from natural source.

**(i) Preparation of methanolic extract**

Plant material *Datura metel,* (Solanaceae) was collected during the month of April/May from near about the railway station Kishanganj; Delhi, India. Hamdard College of Pharmacy, New Delhi, authenticated the identification of plant material where voucher sample has been preserved. The freshly collected plant material was dried in the shade in a well-ventilated enclosure. Dried material was powdered and extracted with methanol. All the extracts obtained after four cycles of extraction with methanol were pooled and evaporated to dryness under reduced pressure at 45 °C in Rotavapor R-114 (Buchi) attached to a Waterbath B-480 (Buchi). The residue obtained was stored at 4°C till use. Methanolic extract was examined for its antifungal activity against species of *Aspergillus*. The extract having antifungal activity was subjected to further fractionation for isolation of the active antifungal component

### *(ii)* Fractionation of methanolic extract of *Datura metel*

The constituents of crude methanolic preparation were fractionated by the method of Harbourne, with slight modifications (Harbourne J.B., 1997, Phytochemical Methods, Chapman and Hall, London, pp. 1-5). The Methanolic extract was successively extracted with hexane, chloroform and acetone respectively. The methanolic extract was first extracted with hexane four times. All the four extracts of hexane were pooled and solvent was evaporated with the help of Rotavapour R-114 (Buchi). The dry fraction was collected and kept at 4°C till further use. The residue after the extraction with hexane was extracted stepwise with chloroform and acetone four times in each case to obtain chloroform and acetone fractions. The leftover residue was finally dissolved in methanol. Solvents were evaporated and dry fractions were recovered. All the fractions were labeled properly and examined for their antifungal activity using pathogenic species of *Aspergillus.* The chloroform fraction was found to be active, therefore, it was further sub-fractionated by column and thin layer chromatography.

### (iii) Column Chromatography

The chloroform fraction was sub-fractionated by modified column chromatography using a silica gel column. Silica gel was suspended in hexane and packed in a glass column of 1.25x35 cm size. Slurry of chloroform extract was prepared and loaded on to the top of the pre-equilibrated silica gel (60-120 $\mu$) column. The components of chloroform fraction were eluted with 100 ml of chloroform at a flow rate of 1.0 ml/min followed by different ratio of chloroform: methanol ranging from 100:0 to 0:100. All the sub-fractions were analyzed by TLC. The sub-fractions showing similar profile of Rf values were pooled and dried *in vacuo.* The antifungal activity of each sub-fraction was tested using pathogenic strains of Aspergilli. The sub-fractions, which showed antifungal potential, were further subjected to the thin layer chromatography for identifying and separating out pure active component.

### (iv) Thin layer chromatography (TLC)

The active antifungal column fractions were spotted onto the Silica gel plates (E. Merck Cat No. 1.05554, $F_{254}$) and subjected to TLC with three different solvent systems i.e. Chloroform: Acetone: Diethylamine (5.0:4.0:1.0), Chloroform: Methanol: Diethylamine (8.5:1.5:0.1) and Chloroform: Methanol: Formic acid (8.0:2.0:0.1). The bands on the plates were visualized with UV light at 254 nm and 366 nm and by spraying with Chloropalatinate, Dragendorff reagent and Iodine. The components in different bands were scrapped and examined for antifungal activity. The preparative TLC was performed to obtain active component of our interest.

### (v) High pressure liquid chromatography (HPLC)

The purity of the compound PC-1 obtained from preparative TLC was analyzed by HPLC using RP-8 column (Merck). The reverse phase HPLC was performed isocratically with the solvent system 70 parts acetonitrile: 30 parts water. The test samples were passed through a membrane of pore size before loading. 5.0 $\mu$l of the 0.22 $\mu$ filtered sample was loaded on to the pre-equilibrated HPLC column at room temperature. The flow rate was maintained at 1.0 ml/min.

### Characterization studies

### (i) Structure elucidation

Characterization of the compound was carried out using various techniques such as class identification by chemical methods, derivatisation of the compound, melting point of the purified compound, ultraviolet spectrum (Fig.7), infra red spectrum (Fig 8), mass spectroscopy (Fig 9), nuclear magnetic resonance spectrum (Fig 10) and. COSY (Fig 11).

Details of the spectral data obtained with respect to novel compound are as described earlier.

### Antifungal Activity Assays

The antifungal activity of various fractions such as crude, methanolic extract, sub-fractions and purified compound was studied by microbroth dilution, disc diffusion and spore germination inhibition assays using pathogenic strains of fungi.

**(i) Pathogen**

Pathogenic strains of fungi were obtained from the Mycology Department of Vallabhbhai Patel Chest Institute, Delhi. All the strains were grown on Sabouraud dextrose agar at 37°C. The conidia form these cultures were harvested and suspended in Sabouraud maltose broth. The number of conidia was counted using haemocytometer and their number in the suspension was adjusted to as per need of the experiment.

**(ii) Microbroth Dilution Assay**

The antifungal susceptibility of the fungi to various fractions or the purified component was assayed by the microbroth, dilution method (Forthergill A.W. and McGough A.W., 1995, Clin. Microbiol. Procedure Handbook. *In Vitro* susceptibility Testing of Yeast, 5.15.1-5.15.15). The fungal spores were harvested from 96-h cultures and their number adjusted to $1x10^6$ per ml. The Sabouraud Dextrose medium was dissolved in glass double distilled water and autoclaved at 10 psi for 15 min. 90-μl medium was added into the wells of cell culture plates. The different concentrations of the extract, fractions or the purified compound were prepared in duplicate wells and then the wells were inoculated with 10 μl of spore suspension. The plates were incubated at 37°C and examined macroscopically after 48 h for the growth of fungal mycelia. The activity was represented as -ve if growth was there and +ve if medium appeared clear with out any growth of fungi.

**(iii) Disc Diffusion Assay**

The disc diffusion test was performed in 10 cm diameter (Tarsons) radiation sterilized petri plates as per the method described in Indian Pharmacopoeia (Indian Pharmacopoeia, 1996, Appendix 9, p A101-A110). Sabouraud Dextrose agar medium was dissolved in double glass-distilled water and autoclaved at 10 psi for 15 minutes. It was cooled to 45°C and 20.0 ml was poured into each petri dish. $1x10^6$ conidia in 1.0 ml of conidial suspension were prepared in Sabouraud maltose broth and overlaid on the surface of the agar plate. Different concentrations of the samples were impregnated on the sterilized discs measuring 5.0 mm in diameter from Whatman filter paper number. The discs were placed on the surface of the agar plates already inoculated with the fungal spores. The plates were incubated at 37°C and examined at 48 hrs for zone of inhibition, if any, around the disc. The diameter of zone of inhibition was measured with the help of a scale. The concentration, which developed the zone of inhibition of 6.0-mm diameter, was considered as minimum inhibitory concentration. Amphotericin B was used in assay as a standard control drug. An additional control disc without any sample but impregnated with equivalent amount of solvent was also used. The test was repeated three to five times with various test preparations of plant to ascertain activity.

**(iv) Preparation of Standard Curve**

Standard curve was prepared in accordance with the method given in the Indian Pharmacopoeia (Indian Pharmacopoeia, 1996, Appendix 9, p A101-A110).

**(v) Spore Germination Inhibition Assay**

The modified spore germination inhibition assay was performed as described earlier (Sureder P. and Janaiah C., 1987, Ind. J. Expt. Biol., 25, 233-234). The fungal species were grown on Sabouraud dextrose agar plates at 37°C for 96 h. Conidia were harvested from the plates and their homogenous suspension was prepared in the Sabouraud dextrose broth. The conidia were counted and their number in the suspension was adjusted to $1x10^4$ per ml. The standard drug Amphotericin B and test samples were initially dissolved in minimal quantity of dimethylsulfoxide (DMSO) and then diluted with Sabouraud dextrose medium. Various concentrations of the test samples in 90 μl of culture medium were prepared in 96 well flat bottom micro-culture plates (Nunc) by double dilution method. The wells were prepared in triplicates for each concentration. Each well was then inoculated with 10 μl of spore suspension containing $100\pm5$ spores. The plates were incubated at 37°C for 10 h and then examined for spore germination under inverted microscope (Nickon Diphot). The number of germinated and non-germinated spores was counted. The percent spore germination inhibition (PSGI) was calculated using following formula

$$PSGI = 100 - \frac{\text{No. of spores germinated in drug treated well}}{\text{No. of spores germinated in control well}} \times 100$$

***In vitro* toxicity evaluation**

The *in vitro* toxicity of the active molecule was studied by MTT assay using RAW cells (Mossman, T., 1983, J. Immunol. Methods, 65, 55-63).

**Cell culture:**

The stock culture of RAW cells was obtained from National Facility for Cell and Tissue Culture, Pune. Cells were maintained in RPMI - 1640 medium supplemented with glutamine (2.3 gm/L), fetal calf serum (10% v/v) and gentamycin (50.0 mg/L) at 37°C in Nuair IR Autoflow water Jacketed carbon dioxide incubator.

**Sample preparation:**

The stock solution of the compound was prepared by dissolving 2.5 mg of the purified compound in minimum amount of DMSO and then diluted with double distilled water to make the final volume 1.0-ml. The different concentrations ranging from 1250.0 μg/ml to 19.5 μg/ml were tested in the cytotoxicity assay.

**Cell cytotoxicity assay:**

The cells were harvested at the log phase of growth confluency from the flask. The homogeneous suspension of cells was prepared in 2.0 ml of culture medium. The number of cells in the suspension was counted using a hemocytometer and the cell suspension was diluted in such a way so as to obtain $5x10^7$ viable cells per ml. The viability of the cells was checked with trypan blue dye exclusion test. The cytotoxicity assay was performed in 96 wells flat bottom tissue culture plates (Nunc Nunclon). The cells ($5x10^6$) in 100.0 μl volume were seeded into each well. The plate was incubated at 37 °C in atmosphere of 5% (v/v) $CO_2$ for 8 h. The wells were examined microscopically for the formation of monolayer of the cells. Various concentrations ranging from 1250.0 μg/ml to 19.5 μg/ml of the compound PC-1 (metelatropinyl ester) were added to the monolayer of cells. In +ve control wells, a known cytotoxic protein obtained from *A. fumigatus,* was used. The $ZnSO_4$ (8.0 mg/L) was used as another +ve toxic material in another set of wells. In negative control wells equivalent amount of the solvent was used. The duplicate wells were used for each concentration of the test compound. The plates were incubated at 37°C in 5% (v/v) $CO_2$ incubator overnight (12 h). The medium along with floating dead cells from the wells was removed by inverting the plate. Thus, only the live cells sticking to the surface of plate were left in the wells. The cells were stained with 40.0 μl of 2.5 % dye MTT. After adding the dye to the wells, the plate was kept at 37 °C in 5% $CO_2$ for 1 h. The tissue culture plate was removed from the incubator and all the dye was aspirated. Only live cells took up the dye. The cells were lysed by adding 100.0 μl of isopropanol- HCl to the wells. After lysis of the cells, the plates were read at 540 nm using plate reader (Spectra Max 190, Molecular Device).

Interpretation of results:

The toxicity of the compound was expressed as percentage with respect to that obtained in -ve control sample. The percent cytotoxicity was calculated using the formula given below.

$$\%\ \text{Cytotoxicity} = \frac{\text{OD in -ve control - OD in the test}}{\text{OD in -ve control}} \times 100$$

***In vivo* Efficacy of PC-1**

*In vivo* efficacy of the compound against *A. fumigatus* infection was studied in the Balb/C mice of 6-8 weak of age of either sex, weighing 15-20 g each. The mice model of aspergillosis described by Dixon et al (1989), was used to

study the effects of compound on *in vivo* infection of *A. fumigatus.*

The animals were housed in the micro-barrier cages on sterile bedding and fed *ad libitum* water and food. The mice were divided into 6 groups and each group contained 8-10 animals. The mice were infected experimentally with *A. fumigatus.* Three days prior to infection with conidia, mice were injected subcutaneously with 3 doses (250.0 mg/kg/day) of cortisone acetate in 400.0 μl of PBS. On the infection day, each mouse received $2x10^7$ conidia by nasal instillation of a single droplet of conidial suspension. The animals of this model developed invasive aspergillosis and all the infected animals died within 4-6 days.

**Inoculum prepration for *A. fumigatus* for experimental infection to mice:**

*A. fumigatus* (190/96) isolate was grown on Sabouraud dextrose agar plates at 37 °C for 4 days. The conidia were collected from the culture plates using PBS (pH 7.2) containing 0.05% Tween 80 (Sigma Chemicals) and the suspension was filtered through sterile glass wool. The conidia were pelleted by centrifugation at 2000 rpm and re-suspended in PBS, pH 7.2. The number of conidia, was counted and adjusted to $1x10^8$ conidia/ml. The viability of the conidia was determined by plating the dilutions of suspensions on Sabouraud dextrose agar.

The purified compound (metelatropinyl ester) isolated from the *D. metel* was administered orally in the concentration ranging from 0.0, 25.0 50.0 100.0 200.0 and 400.0 mg/kg body weight to the mice infected experimentally with *A. fumigatus.*

Treatment:

The dosing of the animals (cortisone treated and challenged) with compound in 6 different groups was initiated within 30 min after challenge with *A. fumigatus* conidia.

Group I: The mice were treated orally with 7 doses of 25.0 mg/kg/day of the compound.

Group II: The animals infected with *A. fumigatus* conidia were given 50.0 mg/kg/day of the compound for 7 days orally.

Group III: The animals in this group were given 7 oral doses of 100.0 mg/kg/day of the compound.

Group IV: In this group of animals, 7 oral doses of the compound (200.0 mg/kg/day) were given.

Group V: The animals of this group were treated with 7 doses of 400.0 mg/kg/day orally over a period of 7 days.

Group VI: The animals of this group acted as control and were treated orally with 7 doses of 400 μl PBS containing solvent.

Survival Rate:

The animals were housed in properly labeled cages and kept under close watch for weight loss and the mortality. The survival rate over a period of 10 days was calculated and the fungal burden in survivors determined (Clemons and Stevens, 1994). Of the animals treated with doses of 25.0, 50.0, 100.0, 200.0 and 400.0 mg/kg body weight, 1 out of 10 (10%), 1 out of 8 (12.5%), 3 out of 9 (33.3%), 4 out of 9 (44.4%) and 8 out of 10 (80%) respectively, survived up to $10^{th}$ day.

Quantification of Colony Forming Units:

The mice were kept under constant watch and those getting moribund, dying or survived up to 10 days were sacrificed. The autopsy was performed on the mice who had died to remove their organs for quantification of colony forming units (CFU). The lungs, livers and kidneys of the mice were removed aseptically, placed in sterile PBS (pH 7.2) and homogenized with teflon pestle mortar. The CFU in the animals were determined by plating 10 fold dilutions of organ homogenates on Sabouraud dextrose agar containing 0.05% triton X-100. The triton X-100 limited the colony size and thus greatly facilitated colony counting (Frosco, 1992). After an incubation for 48 h at 37 °C, colonies were counted and results were expressed as CFU per organ. The survival time and CFU indicating the fungal burden in various organs were considered

to evaluate the protective efficacy of the compound *in vivo*

The following examples are given by way of illustration of the present invention and should not be construed to limit of the scope of the present invention.

**Example 1**

**Extraction and fractionation**

Aerial parts of the plant *Datura metel* (Solanaceae) were collected during the month of April from an area around Delhi, India. Hamdard College of Pharmacy, New Delhi, authenticated the identification of plant material where voucher sample has been preserved. The freshly collected plant material was dried in the shade in a well-ventilated enclosure. 400 gm dried material was powdered and extracted with 500 ml methanol in each cycle. All the extracts obtained after four cycles of extraction were pooled and evaporated to dryness under reduced pressure. The 25 gm residue was obtained and stored at 4°C till use.

The methanolic extract was successively extracted with hexane, chloroform and acetone. The methanolic extract was first extracted with hexane four times. All the four extracts of hexane were pooled and solvent was evaporated. The dry hexane fraction was collected and kept at 4°C till further use. The residue after the extraction with hexane was extracted stepwise with chloroform and acetone four times in each case to obtain chloroform and acetone fractions respectively. The leftover residue was finally dissolved in methanol. Dried fractions were examined for their antifungal activity. The fractions, which did not inhibit the growth of *Aspergillus* up to a concentration of 1500 μg/ml and 50 μg/disc in spore germination inhibition and disc diffusion assays respectively, were considered to be non-active. The chloroform fraction of *Datura metel* was found to be active as a concentration of 1250 μg/ml inhibited the germination of 100% of the spores in spore germination inhibition assay and a concentration of 25.0 μg/disc produced the zone of inhibition of activity significance. Therefore, this fraction was further used to identify and purify the active component using various chromatographic methods.

**Example 2**

**Purification of active compound**

The chloroform fraction was sub-fractionated by modified column chromatography as described above. The above said fractions were analyzed by TLC and those showing similar spots were pooled and dried *in vacuo* to get sub-fractions. Total 15 sub-fractions were obtained. The antifungal activity of each sub-fraction thus obtained was tested using pathogenic strains of *Aspergillus.* The sub-fraction numbers 10 and 11 showed potential antifungal activity and they were further subjected to preparative thin layer chromatography for separating the pure active component. Silica gel plates (Cat No. 1.05554, $F_{254}$) were purchased from E. Merck and used for performing TLC. The column chromatographic active sub-fractions were spotted onto the plates and subjected to run with three different solvent systems i.e. chloroform: acetone: diethylamine (5.0:4.0:1.0), chloroform: methanol: diethylamine (8.5:1.5:0.1) and chloroform: methanol: formic acid (8.0:2.0:0.1). The bands on the plates were visualized with UV light at 254 nm and 366 nm and by spraying with chloropalatinate, Dragendorff reagent and iodine. The components in three different major bands resolved with a solvent system chloroform: methanol: formic acid (8.0:2.0:0.1) (Fig 1) having Rf values 0.14, 0.22 and 0.37 respectively were scrapped and examined for antifungal activity. The compound, which had efficacy against *Aspergillus,* was recovered from a band having Rf value of 0.22. The compound was purified by repeated preparative TLC and tested for its antifungal activity. The active compound reacted positively with Dragendorffs reagent showing thereby its alkaloidal nature.

The purity of the compound obtained from preparative TLC was analyzed by HPLC using RP-8 column (Merck). The reverse phase HPLC was performed isocratically with the solvent system acetonitrile: water (70:30). Samples were passed through a membrane of 0.22 μ pore size before loading. A total of 5.0 μl of the sample was loaded on to the pre-equilibrated column at an ambient temperature. The flow rate was maintained at 1.0 ml/min. The HPLC profile of compound purified by TLC showed two peaks, peak-I and II, which had retention times 1.91 mts. and 2.71 mts. respectively (Fig 2). These two corresponding peaks at retention time 1.88 mts. and 2.84 mts respectively were present in the HPLC profile of active sub-fraction obtained from the column chromatography also. The active column sub-fraction had five major peaks corresponding to retention time 1.31, 1.88, 2.84, 3.82 and 4.35 mts respectively. Changing the solvent system in TLC carried out the further purification of partially purified compound. The solvent system comprised of chloroform: methanol: diethylamine (8.5:1.5:0.1) resolved the mixture containing active molecule having $R_f$ at 0.42 (Fig 3). The retention time of compound in HPLC was found to be around 1.9 mts. and it was showing purity from 97% to 100% in various runs (Fig 4). The retention time of the compound remained same in both chromatographic active sub-fraction and its purified form.

### Example 3

**Antifungal activity of pure compound by microbroth dilution assay**

The antifungal susceptibility of the fungi to the purified compound was assayed by the microbroth dilution method. The different concentrations of the purified compound ranging from 21.87 to 350 μg/ml were prepared in duplicate wells by twofold dilution method. The test was done as described above. The antifungal activity of the compound is given in the Table 1. The MIC of the compound was found to be 87.5 μg/ml. At this concentration of compound none the three species of *Aspergillus* showed any visual growth in wells.

Table 1: Activity of the compound against *Aspergillus* sp. after 48 hrs.

| Concentration of compound (μg/ml) | *A. fumigatus* | *A. niger* | *A.flavus* |
|---|---|---|---|
| 350.00 | + | + | + |
| 175.00 | + | + | + |
| 87.50 | + | + | + |
| 43.75 | - | - | - |
| 21.87 | - | - | - |

### Example 4

**Antifungal activity of pure compound by disc diffusion assay**

The disc diffusion test was performed as per described in the section of details of invention. Amphotericin B 1.25 μg/disc was used in assay as positive control standard drug. Additional discs impregnated with equivalent amount of solvent were also used in the assay as negative controls (Fig: 5). The MIC of the compound was observed to be 5 μg/disc, since this concentration developed zone of inhibition having mean diameter of 6.25, 6.15 and 6.4 mm against *A.fumigatus, A. flavus* and *A. niger* respectively (Table 2).

**Table 2: Antifungal activity of compound by disc diffusion method**

| Concentration. (μg/disc) | Diameter of zone of inhibition (mm) | | |
|---|---|---|---|
| | *A.fumigatus* | *A. flavus* | *A.niger* |
| 20.0 | 9.25 | 9.00 | 9:40 |
| 10.0 | 8.50 | 8.10 | 8.60 |
| 5.0 | 6.25 | 6.15 | 6.40 |
| 2.5 | -- | -- | -- |
| Solvent control | -- | -- | -- |

### Example 5

**Comparison of antifungal activity of compound with that of amphotericin B**

The potency of the compound was determined with the help of standard curve constructed using amphotericin B as standard drug against *A.fumigatus.* Standard curve was prepared according to method given in the Indian Pharmacopoeia. The corrected values of diameter of zone of inhibition along with lowest and highest values were determined. These values were plotted on semilog paper to prepare standard curve. The value of diameter of zone of inhibition obtained for compound was put into the standard curve and corresponding dose producing the effect equivalent to amphotericin B was determined to find out the potency of the compound (Fig: 6).
A concentration of 10 μg/disc of the compound produced a diameter of zone of inhibition equivalent to that produced by 2.67 μg of amphotericin B. These observation showed that the compound was 3.74 time less potent as compared to amphotericin B, but more importantly it is a novel lead molecule.

**Example 6**

**Antifungal activity of pure compound by spore germination inhibition assay**

The spore germination inhibition assay was performed to evaluate the activity of the compound. Various concentrations of the compound ranging from 21.875 to 700 μg/ml in 90 μl of the culture medium were prepared by double dilution method. Wells were inoculated with 10 μl of spore suspension and incubated at 37° C for 10 hrs. The number of germinated and non-germinated spores was counted and the percent spore germination inhibition (PSGI) was calculated. The percent spore germination decrease with increased dose of the compound. The MIC of the compound was found to be 87.5 μg/ml (Fig 7).

**Example 7**

**Cell Cytotoxicity**

The *in vitro* cell cytotoxicity of the compound PC-1 was investigated by incubating RAW cells with varying concentrations of PC-1 and measuring the extent of cytotoxicity by MTT assay as described above. A concentration of compound up to 312.5 μg/ml was completely non-toxic to the cells. The higher doses of the compound developed variable toxicity. A concentration of 1250.0 μg/ml of the compound was toxic to 75.95% of the cells. The percent cytotoxicity was plotted against log concentration of the compound to find out concentration which was cytotoxic to 50% of the cells (CT so). The CT $_{50}$ of the compound was found to be 889.2 μg/ml (Fig 12).

**Example 8**

Structure elucidation of the compound

Correlating all the spectral and chemical analysis information of the compound, the applicants have carried out the characterization of the compound.

**(i) UV spectroscopic data**

The compound PC-1, named metelatropinyl ester, showed two maxima in the UV spectrum, which had λ max 300 and 206 nm and absorbance 0.250 and 1.676 respectively. The prominent maxima at 206 nm in UV spectrum might be due to the presence of ester group. The λmax for ketone has been shown to be 208 nm or little higher. Therefore, the peak at 206 nm suggested the presence of ester group in the PC-1.

**(ii) IR spectroscopic data**

The IR spectrum exhibited absorption bands for amino group (3434 $cm^{-1}$), ester group (1711, 1230 $cm^{-1}$), and unsaturation at 1646 $cm^{-1}$ (Fig 8).

**(iii) Mass spectroscopic data**

The molecular mass of the compound was found to be 239.2 on the basis of its FAB mass spectrum and the intensity of the peak was found to be 20%. The calculations made on the basis of peak intensity at m/z 239.2 (20) in the mass spectrum revealed the presence of 13.8 (= 14) carbon atoms in the molecule PC-1 (Furniss et al, 1989; Kemp, 1991). The chemical analysis and mass spectrum of PC-1 indicated the presence of at least one nitrogen and two oxygen atoms in the compound. The chemical formula thus derived for the compound PC-1 turned out to be $C_{14}H_{25}O_2N$. The nitrogen rule also supported the presence of single nitrogen atom in the molecule. The molecular weight derived from the chemical formula was found 239.188, which indeed was the same as shown by the mass spectrum. The mass spectrum of compound displayed the base peak at m/z 58 generated due to $C_1$-$C_2$ and OC-O fission, suggesting secondary nature of the tropane ring. A prominent ion peak at m/z 102 arose due to formation of pentanoic acid moiety ($C_5H_{10}O_2^+$). Cleavage of $C_{1'}$-$C_{2'}$ linkage resulted in the formation of an ion peak at m/z 57 ($C_4H_9^+$) (Fig 9).

**(iv) NMR spectroscopic data**

The $^1$HNMR spectrum displayed multiplets al. δ 4.15 and 3.75 assigned to -C<u>H</u>-O- and -N-C<u>H</u> protons respectively. A double doublet at δ 3.17 assigned to -N_C<u>H</u> protons. A triplet at 3.17 assigned to -N-C$\underline{H}_2$ protons. A triplet at

δ 2.31 and a multiplet at δ 1.95 of one proton each is assigned to -C-C$\underline{H}_2$ protons. A broad six proton signal at δ 1.62 is attributed to $-CH_3$ group protons attached at positions 3 and 4 of dihydropyrrol ring. Two doublet of one proton each at δ 1.35 and 1.42 are assigned to -C$\underline{H}_2$ proton attached to 2-position of dihydropyrrol ring. A seven proton broad signal is assigned to 2x-C$\underline{H}_2$ (each proton of pentanoic side chain, -C$\underline{H}_3$ (three protons) of -CO-CH-C$\underline{H}_3$ group and a triplet at δ 0.87 for three protons is assigned to terminal $-CH_3$ group of pentanoil acid. The absence of any signal beyond δ 4.15 suggested tetrasubstituted olefinic linkage in the molecule. The existence of a methyl group linked to endo-nitrogen atom was resulted out due to absence of any signal between δ 2.0 and δ 3.0 in the $^1$H NMR spectrum. In $^1$H-$^1$H 2D COSY spectrum the correlation between hydrogen atoms of the dihydropyrrol ring was established (Fig 11).

Based on these evidences the structure of the molecule has been elucidated as 2-(3,4-dimethyl-2,5-dihydro-1H-pyrrol-2-yl)-1-methylethyl pentanoate.

The molecule PC-1 identified in the present investigation as 2-(3,4-dimethyl-2,5-dihydro-1H-pyrrol-2-yl)-1 -methylethyl pentanoate is a new tropine derivative and the occurrence of this molecule is being reported for the first time from a plant or synthetic source. This is a novel antifungal compound useful as a lead molecule for the development of new antimycotic drugs. The $CT_{50}$ of amphotericin B and its liposomal derivatives (drug of choice) was reported to be in the range of 4.0-64.0 μg/ml (Zager, R.A., 2000, Am. J. Kidney Dis., 2, 238). The $CT_{50}$ value of novel compound is 889.2 μg/ml which indicated the compound to be many fold less cytotoxic than the amphotericin B.

**Example 9**

***In vivo* Efficacy**

The Balb/C mice of 6-8 weak of age of either sex, weighing 15-20 g, were housed in micro-barrier cages on sterile bedding and fed *ad libitum* water and food. The animals were divided into 6 groups and each group contained 8-10 mice. The efficacy of PC-1 against aspergillosis was studied at five dose levels. Various doses of the compound were administered orally to the animals already challenged with $2x10^7$ spores of *A. fumigatus* shows the survival rate of the animals treated with compound. Of the animals treated with doses of 25.0, 50.0, 100.0, 200.0 and 400.0 mg/kg body weight, 1 out of 10 (10%), 1 out of 8 (12.5%), 3 out of 9 (33.3%), 4 out of 9 (44.4%) and 8 out of 10 (80%) respectively, survived up to 10$^{th}$ day.

The percent survival rate in treated animals increased with the dose, indicating thereby the protective efficacy to be dose dependent. The probit values of percent survival were plotted against log dose of the compound PC-1 on the semilog paper to determine the effective dose required to confer protection in 50% of the animals ($ED_{50}$). The $ED_{50}$ of the compound PC-1 was found to be 167.0 mg/kg body weight (Fig. 13 ).

**Example 10**

**Quantification of Colony Forming Units**

The mice were kept under constant watch and those getting moribund, dying or survived up to 10 days were sacrificed. The autopsy was performed on the mice who had died to remove their organs for quantification of colony forming units (CFU). The lungs, livers and kidneys of the mice were removed aseptically, placed in sterile PBS (pH 7.2) and homogenized with teflon pestle mortar. The CFU in the animals were determined by plating 10 fold dilutions of organ homogenates on Sabouraud dextrose agar containing 0.05% triton X-100. The triton X-100 limited the colony size and thus greatly facilitated colony counting (Frosco, 1992). After an incubation for 48 h at 37 °C, colonies were counted and results were expressed as CFU per organ.

The survival time and CFU indicating the fungal burden in various organs were considered to evaluate the protective *in vivo* efficacy of the compound 2-(3,4-dimethyl-2,5-dihydro-1H-pyrrol-2-yl)-1-methylethyl pentanoate.

The fungal burden in the organs of the animals, which died or became moribund during study or those survived up to 10$^{th}$ day, was determined. The lungs, livers and kidneys of the animals were isolated, homogenized and the homogenate was used to culture the pathogen on Sabouraud dextrose agar plates. The colonies of the fungus were counted and expressed as colony forming units (CFU)/organ. Table 3 shows the number of CFU in different organs.

The doses of 25.0 and 50.0 mg/kg body weight did not have any effect on the CFUs in the lung tissue. However, protective effect was observed in animals treated with higher doses. A dose of 400 mg/kg body weight of PC-1, reduced the colony counts significantly as compared to controls. The difference was found to be statistically significant ($p < 0.001$). The liver was found to have least burden of fungus and there was very less effect of the compound on CFUs in liver. The kidneys appeared to be more susceptible to the infection as the highest number of CFUs was detected in the kidneys of control animals and those treated with 25.0 or 50.0 mg/kg body weight of the compound. However, there was significant reduction in the number of CFUs in those animals which were treated with 100.0 to 400.0 mg/kg body weight of the purified antifungal compound 2-(3,4-dimethyl-2,5-dihydro-1H-pyrrol-2-yl)-1-methylethyl pentanoate (Table 3).

**Table 3**

| Colony forming units in mice treated with various doses of the purified antifungal compound 2-(3,4-dimethyl-2,5-dihydro-1H-pyrrol-2-yl)-1-methylethyl pentanoate | | | | |
|---|---|---|---|---|
| Groups | Dose (mg/kg body weight) | CFU/Organs (Mean+ SD) | | |
| | | Lung | Liver | Kidney |
| I | 25.0 | 907.5+246.09 | 255.0+72.45 | 1402.5+350.09 |
| II | 50.0 | 900.0+230.29 | 290.6+135.58 | 1040.5+441.78 |
| III | 100.0 | 650.1+171.85 | 216.7+108.97 | 733.3+394.69 |
| IV | 200.0 | 525.0+251.56 | 225.0+91.85 | 683.3+352.67 |
| V | 400.0 | 180.0+127.75 | 165.0+117.84 | 225.0+157.39 |
| Control | 0.0 | 877.5+276.25 | 307.5+159.88 | 1440.0+439.89 |

The main advantages of the present invention are

(i) Present invention provides a novel antifungal lead compound.

(ii) This novel molecule can be used to develop new drugs for treating various fungal diseases of humans.

(iii) Invention also provides a method for isolation, purification and characterisation of the novel antifungal compound from a plant *Datura metel.*

(iv) The novel molecule is less toxic than some of the available antifungal drugs in the market.

## Claims

**1.** A novel antifungal molecule 2-(3,4-dimethyl-2,5-dihydro-1H-pyrrol-2-yl)-1-methylethyl pentanoate of formula 1

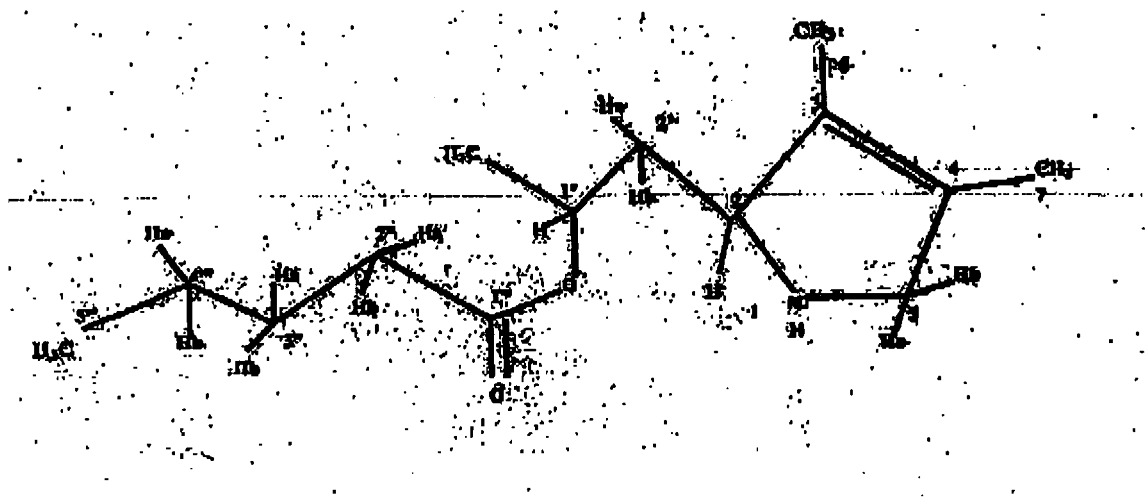

(I)

**2.** A novel antifungal molecule as claimed in claim 1, wherein the said hetrocyclic molecule is **characterized by** known analytical methods such as TLC. group specific chemical staining, UV, infra-red, mass and nuclear magnetic resonance spectroscopy and biological assays such as anti-mycotic assay and cytotoxicity assay and has following characteristics :

a) $R_f$ value of 0.22 on TLC as shown in Fig 3 of accompanying drawings,
b) Absorption maixma at 300 nm and 206 nm in UV spectrum as shown in Fig.7 of accompanying drawings,
c) IR $V_{Max}$ 3434 (NH), 3024, 1711 (ester), 1646 (C=C), 1524, 1426,1230, 938, 760 $cm^{-1}$ as shown in Fig 8 of accompanying drawings,
d) +ve FABMS m/z: 239 $[M]^+$ ($C_{14} H_{25} O_2$ N) (16.3), 212 (13.2), 174 (16.2), 122 (34.2), 102 (96.4), 58 (100), and 57 (19.2) as shown in Fig 9 of accompanying drawings, and
e) $^1H$ NMR ($CDCl_3$): δ 4.15 (1H, m, $C_1$-H); 3.75 (1H, m, $C_2$-H); 3.17 (2H, dd, J=7.44 Hz, 7.44 Hz, $C_5$-$H_a$ and $C_5$-$H_a$ and $C_5$-$H_b$); 2.31 (1H,t, J=6,48 Hz, $C_2$-$H_a$); 1.95 (1H, m, $C_2$- -$H_b$); 1.62 (6H, br, $C_6$-$CH_3$ and $C_7$-$CH_3$); 1.42 (1H, d, J-6.96 Hz, $C_2$-$H_a$); 1.35 (1H, d, J=7.28 Hz, $C_{2'}$-$H_b$); 1.25 (7H, br, $C_{1'}$-$CH_3$, $C_{3''}$ -$H_b$, $C_{4''}$-$H_a$ and $C_{4''}$ -$H_b$); 0.87 (3H, t, J=5.96 Hz, $C_{5''}$-$CH_3$) and COSY spectrum as shown in figure 11 of accompanying drawings.

**3.** The compound as claimed in claim 1, wherein the $CT_{50}$ value (i.e. cytotoxic dose to 50% of the cells) of the said antifungal molecule is 889.2 μg/ml, as shown in Fig 12 of the accompanying drawings.

**4.** The compound as claimed in claim 1, wherein $ED_{50}$ for the said antifungal compound is 167.0 mg/kg body weight, as shown in Fig 13 of accompanying drawings.

**5.** The compound as claimed in claim 1, wherein cytotoxicity of said compound is 57.8 times less than the standard antifungal drug

**6.** The compound as claimed in claim 1, wherein the said compound is used as an antifungal agent.

**7.** A pharmaceutical composition as an antifungal agent, said composition comprising effective amount of compound 2-(3,4-dimethyl-2,5-dihydro-1H-pyrrol-2-yl)-1-methylethyl pentanoate along with pharmaceutically acceptable additives and adjuvants.

**8.** The composition as claimed in claim 7, the additives is selected from the group consisting of carbohydrate, protein, sugar and pharmaceutically acceptable carriers.

**9.** The composition as claimed in claim 7, wherein the effective dose of novel antifungal molecule is in the range of 100 to 400 mg/kg body weight

**10.** The composition as claimed in claim 7, wherein said composition may be administered orally, intraperitoreal or any other suitable routes

**11.** The composition as claimed in claim 7, wherein the said composition is administered in the form of tablet, capsule, syrup, powder, ointment and injection.

**12.** The composition as claimed in claim 7, wherein the cytotoxicity of the said composition is 57.8 times less than the standard antifungal drug.

**13.** The composition as claimed in claim 7, wherein the said composition may be administered to subjects selected from mammals and humans preferably humans

**14.** A process for isolation of a novel antifungal molecule as claimed in claim 1, said process comprises the steps of:

(i) extracting successively the powdered *Datura metel* plant material with an organic solvent at a temperature in the range of 15 to 45 °C,
(ii) removing the solvent to obtain residue,
(iii) extracting the above said residue obtained in step (ii) with an aliphatic hydrocarbon solvent followed by extraction with chloroform,
(iv) removing chloroform from chloroform fractions,
(v) screening the above obtained residue from chloroform fractions for antimycotic activity,
(vi) separating the antifungal fractions and purifying the novel antifungal lead molecule from active antimycotic chloroform fractions by conventional chromatographic methods, and
(vii) assaying the lead molecule obtained in step (vi) for antifungal activity and its cytotoxicity.

**15.** A process as claimed in claim 14, wherein in step (i) the solvent used for extraction is selected from methanol,

ethanol, acetone, and chloroform.

**16.** A process as claimed in claim 14, wherein in step (ii) the purification of compound is carried out by column or thin layer chromatography (TLC) and high performance liquid chromatography (HPLC).

**17.** A process as claimed in claim 14, wherein in step (v) the aliphatic hydrocarbon solvent used is selected from pentane, hexane, petroleum ether and heptane.

**18.** A process as claimed in claim 14, wherein in step (vi) the purification of novel compound is carried out by Thin Layer Chromatography using solvent systems selected from Chloroform: Acetone: Diethylamine (5.0:4.0:1.0), Chloroform: Methanol: Diethylamine (8.5:1.5:0.1) and Chloroform: Methanol: Formic acid (8.0:2.0:0.1) or different combinations of above said organic solvents.

**19.** A process as claimed in claims 14, wherein in step (vi) the purification of novel compound is effected by HPLC using solvent system 70:30 of acetonitrile and water using reverse phase RP-8 column.

**20.** A process as claimed in claim 14, wherein in step (vi), the pure compound exhibits antifungal activity

**Patentansprüche**

**1.** Neues antimykotisches Molekül 2-(3,4-Dimethyl-2,5-dihydro-1H-pyrrol-2-yl)-1-methylethylpentanoat der Formel 1

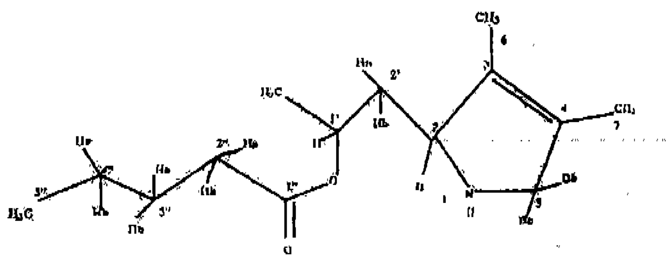

(I)

**2.** Neues antimykotisches Molekül wie in Anspruch 1 beansprucht, wobei das besagte heterocyclische Molekül durch bekannte Analysenverfahren wie DC, gruppenspezifische chemische Anfärbung, UV, Infrarot, Massenspektroskopie und Kernresonanzspektroskopie und biologische Tests wie einen antimykotischen Test und einen Cytotoxizitätstest charakterisiert wird und die folgenden charakteristischen Merkmale aufweist:

a) ein $R_f$-Wert von 0.22 in der DC wie es in Fig. 3 der beigefügten Abbildungen gezeigt ist.
b) Absorptionsmaxima bei 300 nm und 206 nm im UV-Spektrum, wie es in Fig. 7 der beigefügten Abbildungen gezeigt ist.
c) IR $V_{Max}$: 3434 (NH), 3024, 1711 (Ester). 1646 (C=C), 1524, 1426, 1230, 938. 760 $cm^{-1}$, wie es in Fig. 8 der beigefügten Abbildungen gezeigt ist.
d) +ve FABMS m/z: 239 $[M]^+$ ($C_{14}H_{25}O_2N$) (16,3), 212 (13,2), 174 (16,2), 122 (34,2), 102 (96.4). 58 (100) und 57 (19.2), wie es in Fig. 9 der beigefügten Abbildungen gezeigt ist, und
e) $^1$H-NMR ($CDCl_3$): δ 4.15 (1H, m, $C_1$-H); 3,75 (1H, m, $C_2$-H); 3.17 (2H, dd, J=7,44 Hz. 7,44 Hz, $C_5$-$H_a$ und $C_5$-$H_a$ und $C_5$-$H_b$); 2.31 (1H, t, J=6,48 Hz, $C_{2'''}$-$H_a$); 1,95 (1H, m, $C_{2'''}$-$H_b$); 1,62 (6H, br, $C_6$-$CH_3$ und $C_7$-$CH_3$); 1,42 (1H, d, J=6.96 Hz, $C_{2'}$-$H_a$); 1,35 (1H, d, J=7,28 Hz, $C_{2'}$-$H_b$); 1,25 (7H, br, $C_{1'}$-$CH_3$, $C_{3'''}$-$H_b$, $C_{4'''}$-$H_a$ und $C_{4'''}$-$H_b$); 0,87 (3H, t, J=5,96 Hz, $C_{5'''}$-$CH_3$) und ein COSY-Spektrum, wie es in Fig. 11 der beigefügten Abbildungen gezeigt ist.

**3.** Verbindung wie in Anspruch 1 beansprucht, wobei der $CT_{50}$-Wert (d.h. die cytotoxische Dosis für 50 % der Zellen) des besagten antimykotischen Moleküls 889.2 µg/ml beträgt, wie es in Fig. 12 der beigefügten Abbildungen gezeigt ist.

**4.** Verbindung wie in Anspruch 1 beansprucht, wobei die $ED_{50}$ für die besagte antimykotische Verbindung 167,0 mg/kg Körpergewicht beträgt, wie es in Fig. 13 der beigefügten Abbildungen gezeigt ist.

**5.** Verbindung wie in Anspruch 1 beansprucht, wobei die Cytotoxizität der Verbindung 57,8 mal geringer ist als die des antimykotischen Standardarzneimittels.

**6.** Verbindung wie in Anspruch 1 beansprucht, wobei die Verbindung als ein Antimykotikum verwendet wird.

**7.** Pharmazeutische Zusammensetzung als Antimykotikum, wobei die Zusammensetzung eine wirksame Menge der Verbindung 2-(3,4-Dimethyl-2,5-dihydro-1H-pyrrol-2-yl)-1-methylethylpentenoat zusammen mit pharmazeutisch annehmbaren Zusätzen und Adjuvanzien umfasst.

**8.** Zusammensetzung wie in Anspruch 7 beansprucht, wobei die Zusätze ausgewählt sind aus der Gruppe bestehend aus Kohlenhydrat, Protein, Zucker und pharmazeutisch annehmbaren Trägern.

**9.** Zusammensetzung wie in Anspruch 7 beansprucht, wobei die Wirkdosis des neuen antimykotischen Moleküls im Bereich von 100 bis 400 mg/kg Körpergewicht liegt.

**10.** Zusammensetzung wie in Anspruch 7 beansprucht, wobei die Zusammensetzung oral. intraperitoneal oder auf beliebigen anderen geeigneten Wegen verabreicht werden kann.

**11.** Zusammensetzung wie in Anspruch 7 beansprucht, wobei die Zusammensetzung in Form einer Tablette, einer Kapsel, eines Sirups, eines Pulvers, einer Salbe und einer Injektion verabreicht wird.

**12.** Zusammensetzung wie in Anspruch 7 beansprucht, wobei die Cytotoxizität der Zusammensetzung 57.8 mal geringer ist als die des antimykotischen Standardarzneimittels.

**13.** Zusammensetzung wie in Anspruch 7 beansprucht, wobei die Zusammensetzung an Subjekte verabreicht werden kann, die ausgewählt sind aus Säugern und Menschen, vorzugsweise Menschen.

**14.** Verfahren zur Isolierung eines neuen antimykotischen Moleküls wie in Anspruch 1 beansprucht, wobei das Verfahren die folgenden Schritte umfasst:

(i) nacheinander Extrahieren des pulverisierten *Datura metel*-Pflanzenmaterials mit einem organischen Lösungsmittel bei einer Temperatur im Bereich von 15 bis 45 °C.
(ii) Entfernen des Lösungsmittels, um einen Rückstand zu erhalten,
(iii) Extrahieren des vorstehend in Schritt (ii) erhaltenen Rückstands mit einem aliphatischen Kohlenwasserstofflösungsmittel, gefolgt von einer Extraktion mit Chloroform,
(iv) Entfernen von Chloroform aus Chloraformfraktionen,
(v) Durchsuchen (Screening) des vorstehend erhaltenen Rückstands aus Chloroformfraktionen im Hinblick auf eine antimykotische Aktivität,
(vi) Abtrennen der antimykotischen Fraktionen und Reinigen des neuen antimykotischen Leitmoleküls aus aktiven antimykotischen Chloroformfraktionen durch herkömmliche chromatografische Verfahren, und
(vii) Testen des in Schritt (vi) erhaltenen Leitmoleküls im Hinblick auf eine antimykotische Aktivität und seine Cytotoxizität.

**15.** Verfahren wie in Anspruch 14 beansprucht, wobei in Schritt (i) das für die Extraktion verwendete Lösungsmittel ausgewählt ist aus Methanol, Ethanol, Aceton und Chloroform.

**16.** Verfahren wie in Anspruch 14 beansprucht, wobei in Schritt (ii) die Reinigung der Verbindung durch Säulen- oder Dünnschichtchromatographie (DC) und Hochleistungsflüssigchromatografie (HPLC) durchgeführt wird.

**17.** Verfahren wie in Anspruch 14 beansprucht, wobei in Schritt (v) das verwendete aliphatische Kohlenwasserstofflösungsmittel ausgewählt ist aus Pentan, Hexan, Petrolether und Heptan.

**18.** Verfahren wie in Anspruch 14 beansprucht, wobei in Schritt (vi) die Reinigung der neuen Verbindung durch Dünnschichtchromatografie unter Verwendung von Lösungsmittelsystemen ausgewählt aus Chloroform:Aceton:Diethylamin (5,0:4,0:1,0). Chloroform:Methanol Diethylamin (8,5:1,5:0,1) und Chloroform:Methanol:Ameisensäure (8,0:

2,0:0,1) oder verschiedenen Kombinationen dervorstehend,genannten organischen Lösungsmittel durchgeführt wird.

**19.** Verfahren wie in Anspruch 14 beansprucht, wobei in Schritt (vi) die Reinigung der neuen Verbindung durch HPLC unter Verwendung eines Lösungsmittelsystems von 70:30 Acetonitril und Wasser unter Verwendung einer Umkehrphasensäule RP-8 erfolgt.

**20.** Verfahren wie in Anspruch 14 beansprucht, wobei in Schritt (vi) die reine Verbindung eine antimykotische Aktivität aufweist.

**Revendications**

**1.** Nouvelle molécule antifongique, le pentanoate de 2-(3,4-diméthyl-2,5-dihydro-1H-pyrrol-2-yl)-1-méthyléthyle, de formule I

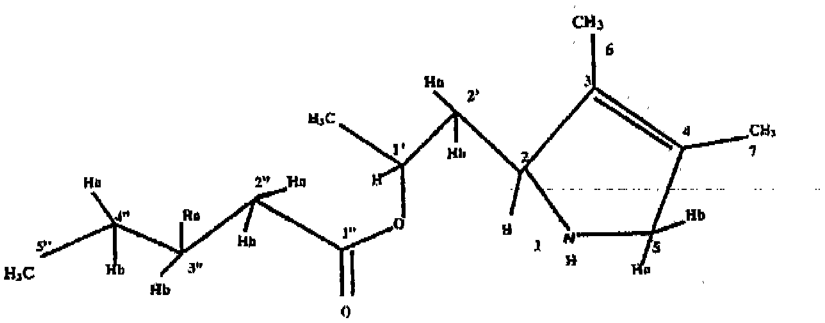

(I)

**2.** Nouvelle molécule antifongique selon la revendication 1, dans laquelle ladite molécule hétérocyclique est **caractérisée par** des procédés analytiques connus, tels que la CCM, la coloration chimique spécifique de groupes, la spectroscopie UV, infra-rouge, de masse et de résonance magnétique nucléaire et des tests biologiques tels que des tests anti-mycotiques et des tests de cytotoxicité, et a les caractéristiques suivantes :

a) valeur de $R_f$ de 0,22 en CCM comme représenté à la figure 3 des dessins accompagnants,
b) maxima d'absorption à 300 nm et 206 nm dans le spectre UV comme représenté à la figure 7 des dessins accompagnants,
c) IR $V_{max}$ 3434 (NH), 3024, 1711 (ester), 1646 (C=C), 1524, 1426, 1230, 938, 760 $cm^{-1}$ comme représenté la figure 8 des dessins accompagnants,
d) +ve FABMS m/z : 239 $[M]^+$ ($C_{14}H_{25}O_2N$) (16,3), 212 (13,2), 174 (16,2), 122 (34,2), 102 (96,4), 58 (100) et 57 (19,2) comme représenté à la figure 9 des dessins accompagnants, et
e) $^1H$ RMN ($CDCl_3$) : $\delta$ 4,15 (1H, m, $C_{1'}$-H); 3,75 (1H, m, $C_2$-H); 3,17 (2H, dd, J=7,44 Hz, 7,44 Hz, $C_5$-$H_a$ et $C_5$-$H_a$ et $C_5$-$H_b$); 2,31 (1H,t, J=6.48 Hz, $C_{2''}$-$H_a$); 1,95 (1H, m, $C_{2''}$-$H_b$; 1,62 (6H, large, $C_6$-$CH_3$ et $C_7$-$CH_3$) ; 1,42 (1H, d, J=6,96 Hz, $C_{2'}$-$H_a$); 1,35 (1H,d,J=7,28 Hz, $C_{2'}$-Hb); 1,25 (7H, large, $C_{1'}$-$CH_3$, $C_{3''}$-Hb, $C_{4''}$-$H_a$ et $C_{4''}$-$H_b$); 0,87 (3H, t, J=5,96 Hz, $C_{5''}$-$CH_3$) et spectre COSY comme représenté à la figure 11 des dessins accompagnants.

**3.** Composé selon la revendication 1, dans lequel la valeur $CT_{50}$ (c'est-à-dire la dose cytotoxique pour 50% des cellules) de ladite molécule antifongique est de 889,2 $\mu$g/ml, comme représenté à la figure 12 des dessins accompagnants.

**4.** Composé selon la revendication 1, dans laquelle la $DE_{50}$ dudit composé antifongique est de 167,0 mg/kg de poids corporel, comme représenté à la figure 13 des dessins accompagnants.

**5.** Composé selon la revendication 1, dans lequel la cytotoxicité dudit composé est 57,8 fois inférieure à celle du médicament antifongique standard.

**6.** Composé selon la revendication 1, dans lequel ledit composé est utilisé comme agent antifongique.

**7.** Composition pharmaceutique comme agent antifongique, ladite composition comprenant une quantité efficace du

composé pentanoate de 2-(3,4-dimëthyl-2,5-dihydro-1H-pyrrol-2-yl)-1-méthyléthyle en même temps que des additifs et adjuvants pharmaceutiquement acceptables.

**8.** Composition selon la revendication 7, les additifs étant sélectionnés dans le groupe consistant en un hydrate de carbone, une protéine, un sucre et des supports pharmaceutiquement acceptables.

**9.** Composition selon la revendication 7, dans laquelle la dose efficace de la nouvelle molécule antifongique est comprise dans la gamme allant de 100 à 400 mg/kg de poids corporel.

**10.** Composition selon la revendication 7, ladite composition pouvant être administrée par voie orale, intrapéritanéale ou toute autre voie convenable.

**11.** Composition selon la revendication 7, ladite composition étant administrée sous la forme d'un comprimé, d'une gélule, d'un sirop, d'une poudre, d'un onguent et d'une forme injectable.

**12.** Composition selon la revendication 7, dans laquelle la cytotoxicité de ladite composition est 57,8 fois inférieure à celle du médicament antifongique standard.

**13.** Composition selon la revendication 7, ladite composition pouvant être administrée à des sujets sélectionnés entre les mammifères et les êtres humains, de préférence les êtres humains.

**14.** Procédé d'isolation d'une nouvelle molécule antifongique selon la revendication 1, ledit procédé comprenant les étapes de :

(i) extraction successive du matériau végétal de *Datura metel* en poudre au moyen d'un solvant organique à une température comprise dans la gamme allant de 15 à 45°C,
(ii) élimination du solvant pour obtenir un résidu,
(iii) extraction du résidu issu de l'étape (ii) ci-dessus à l'aide d'un hydrocarbure aliphatique comme solvant, puis extraction avec du chloroforme,
(iv) élimination du chloroforme des fractions chloroformiques,
(v) criblage, quant à leur activité antimitotique, du résidu obtenu ci-dessus, issu des fractions chloroformiques,
(vi) séparation des fractions antifongiques et purification de la nouvelle molécule amorce antifongique à partir des fractions chloroformiques antimitotiques actives par des méthodes chromatographiques classiques, et
(vii) test de la molécule amorce obtenue à l'étape (vi) quant à son activité antifongique et à sa cytotoxicité.

**15.** Procédé selon la revendication 14, dans lequel, dans l'étape (i), le solvant utilisé pour l'extraction est sélectionné parmi le méthanol, l'éthanol, l'acétone et le chloroforme.

**16.** Procédé selon la revendication 14, dans lequel, dans l'étape (ii), la purification du composé est mise en oeuvre par chromatographie sur colonne ou sur couche mince (CCM) et chromatographie liquide haute performance (CLHP).

**17.** Procédé selon la revendication 14, dans lequel, dans l'étape (v), l'hydrocarbure aliphatique utilisé comme solvant est sélectionné parmi le pentane, l'hexane, l'éther de pétrole et l'heptane.

**18.** Procédé selon la revendication 14, dans lequel, dans l'étape (vi), la purification du nouveau composé est mise en oeuvre par Chromatographie sur Couche Mince et utilisant des systèmes de solvants sélectionnés parmi les mélanges chloroforme:acétone:diéthylamine (5,0:4,0:0,1), chloroforme:méthanol:diéthylamine (8,5:1,5:0,1) et chloroforme:méthanol:acide formique (8,0:2,0:0,1) ou différentes combinaisons desdits solvants organiques.

**19.** Procédé selon la revendication 14, dans lequel, dans l'étape (vi), la purification du nouveau composé est effectuée par CLHP en utilisant un système de solvant acétonitrile:eau dans le rapport 70:30 avec une colonne RP-8 en phase inverse.

**20.** Procédé selon la revendication 14, dans lequel, dans l'étape (vi), le composé pur manifeste une activité antifongique.

Fig 1: TLC profiles of the active column sub-fractions and purified compound.

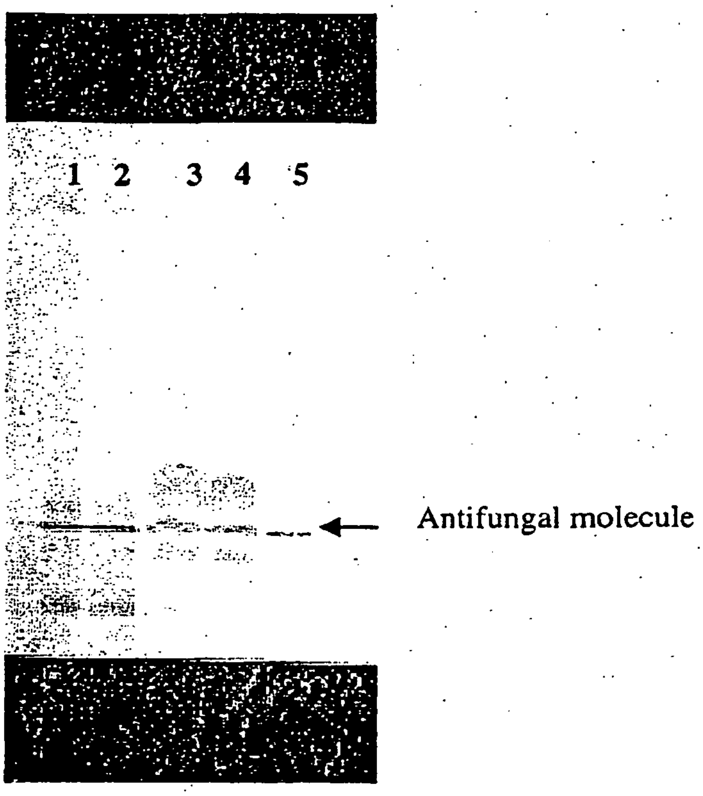


Solvent System: Methanol: Chloroform: Formic acid (8.0: 2.0: 1.0)
Spray: Dragendroff's reagent

Fig 2: HPLC profile of TLC purified antifungal fraction.

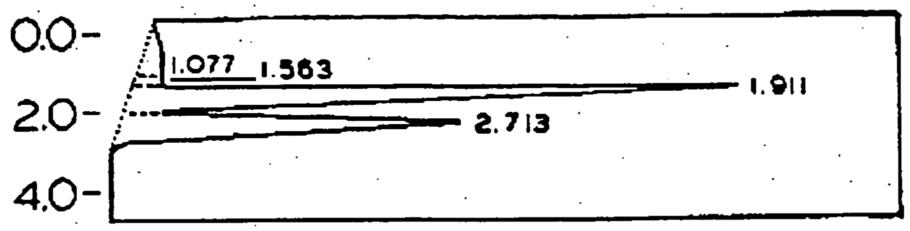


※ Calculation report ※※

| PKNO | TIME | AREA | HEIGHT | CONC. |
|---|---|---|---|---|
| 1 | 1.077 | 156092 | 2627 | 7.5151 |
| 2 | 1.563 | 38151 | 3229 | 1.8368 |
| 3 | 1.911 | 981754 | 68883 | 47.2666 |
| 4 | 2.713 | 901059 | 38171 | 43.3815 |
| | TOTAL | 2077055 | 112910 | 100 |

Fig 3: TLC profile of HPLC purified frcation.

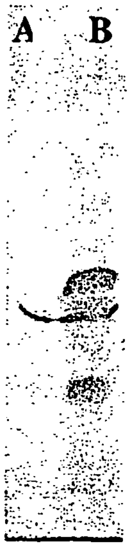


A- Purified Compound
B- Column Sub-fraction

Fig 4: HPLC profile of the purified fraction.

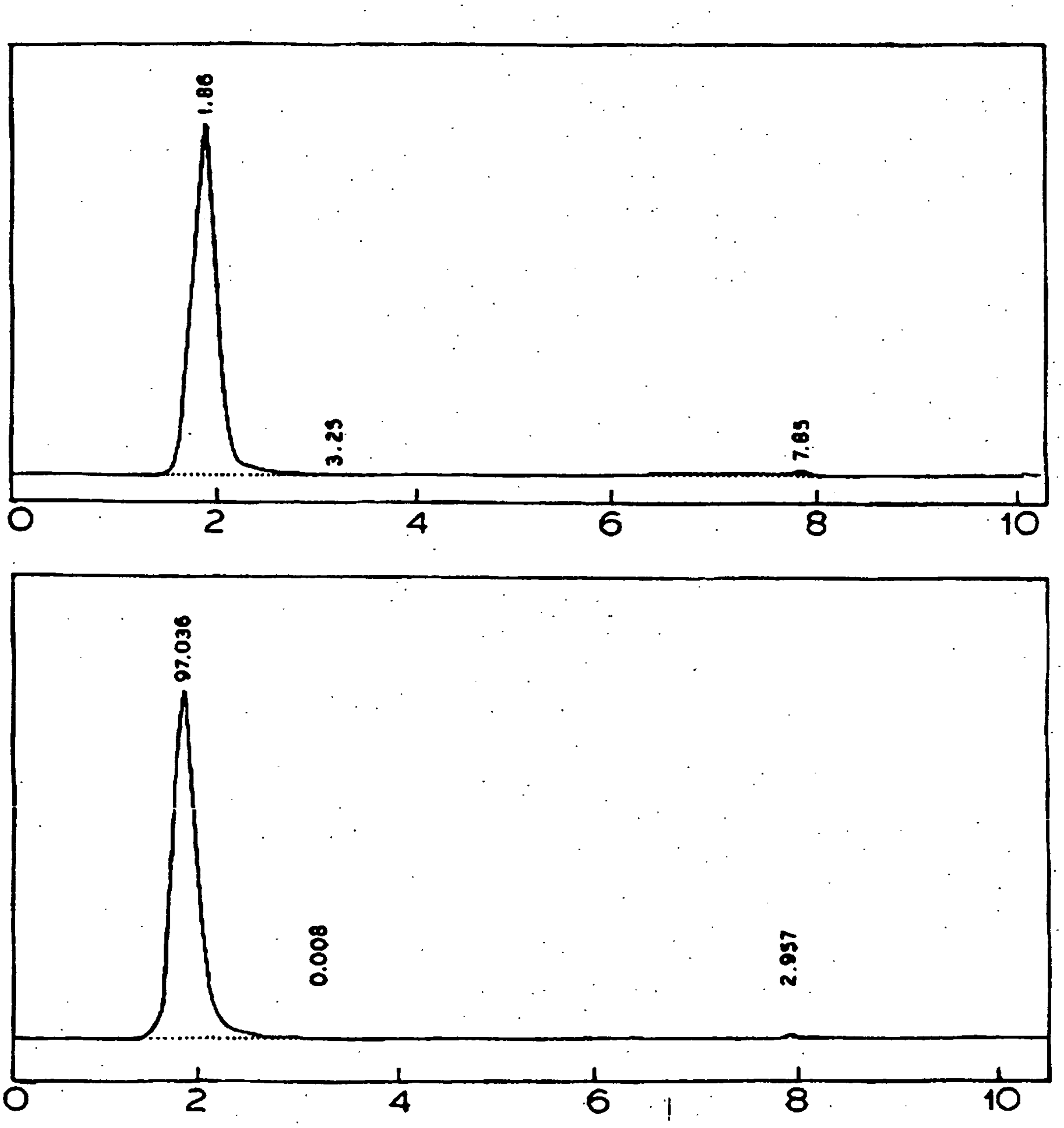

Fig 5: Inhibition of growth of *Aspergillus. fumigatus* by the by disc diffusion method.

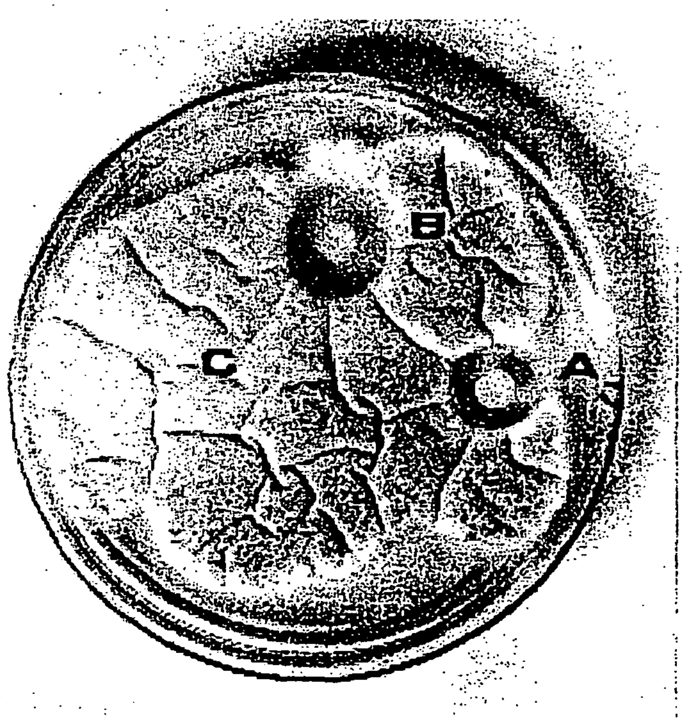


| |
|---|
| A-Test Compound (10 μg/disc)<br>B-Standard drug (2.5 μg/disc)<br>C- Solvent Control |

Fig 6: Percent spore germination inhibition by the compound against *Aspergillus* species.

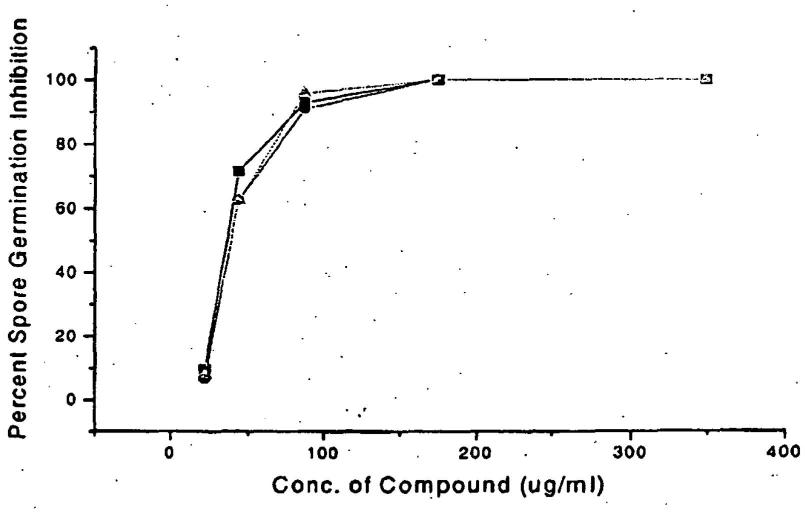

Fig 7: Ultra-Violet spectrum of the compound.

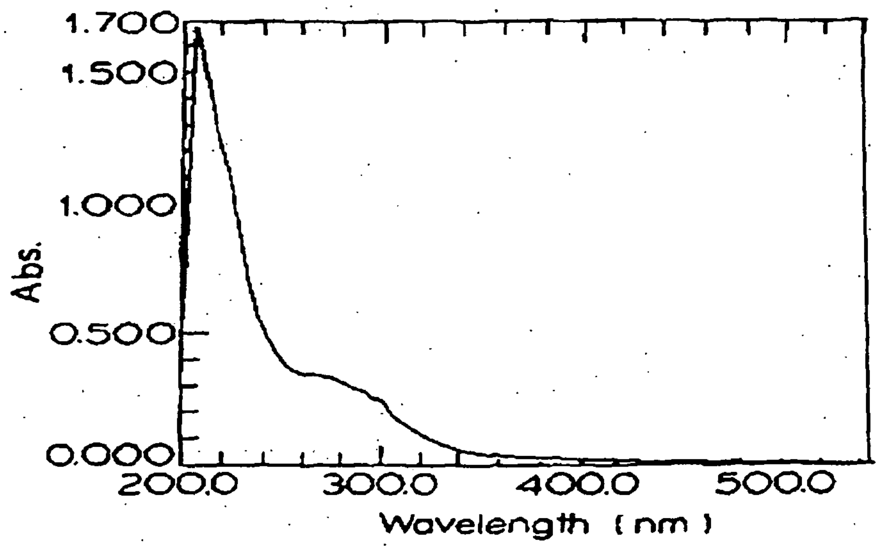


Peak pick

| NO. | WAVELENGTH (nm.) | ABC. |
|---|---|---|
| 1 | 300.00 | 0.250 |
| 2 | 206.50 | 1.676 |

Fig 8: Infra-red spectrum of the purified compound.

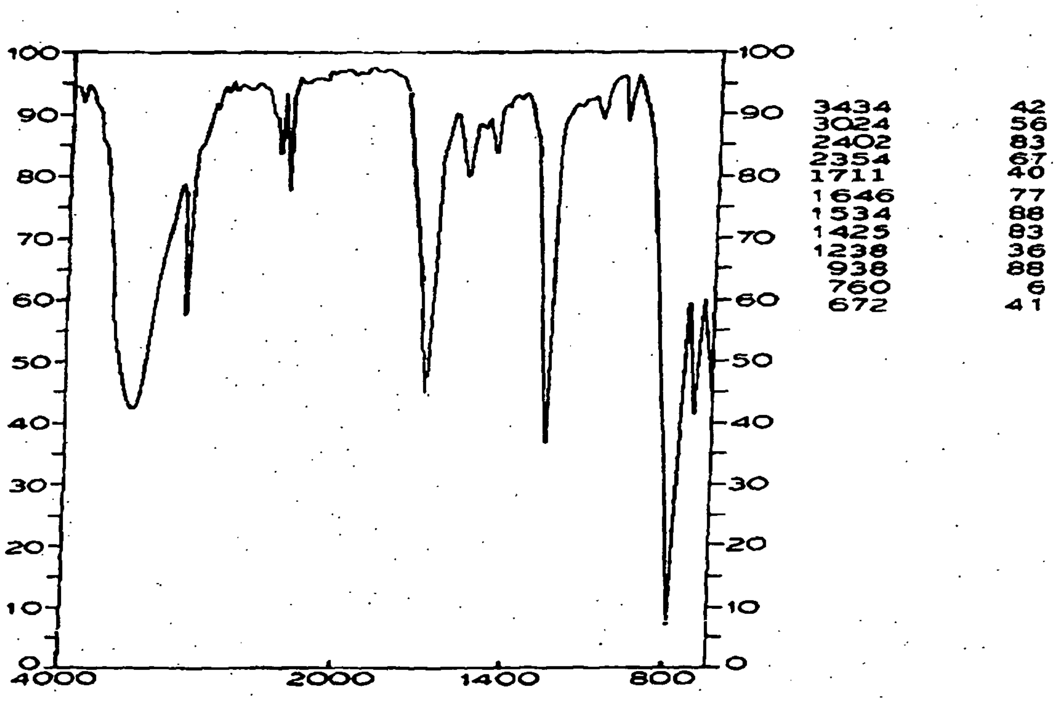

Fig 9: Mass spectrum of the purified compound.

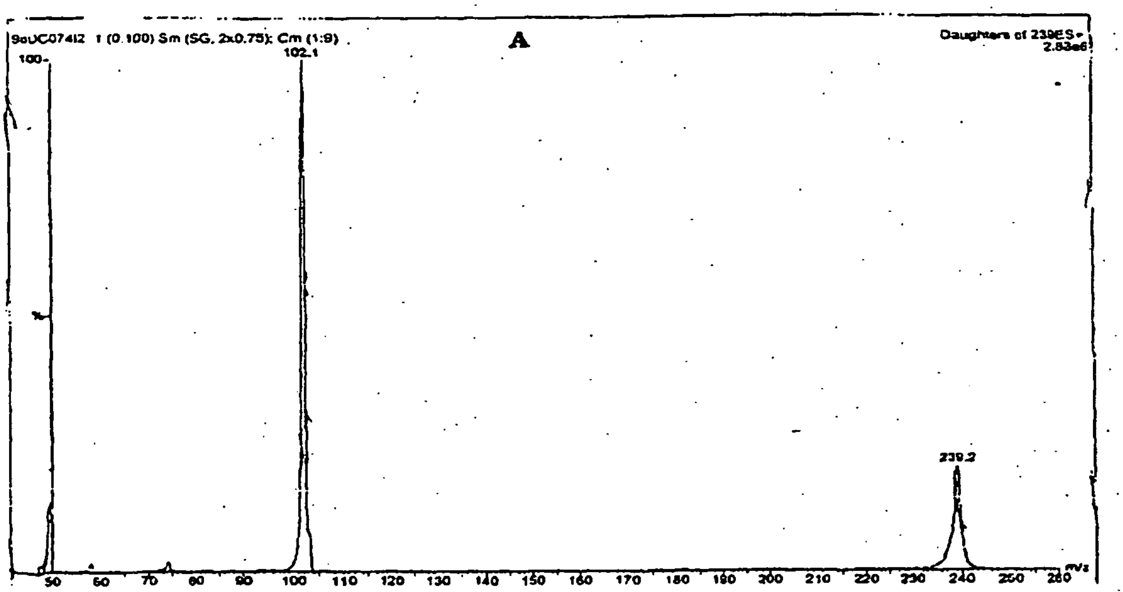

Fig.10. NMR spectrum of the compound (Values in boxes are δ values)

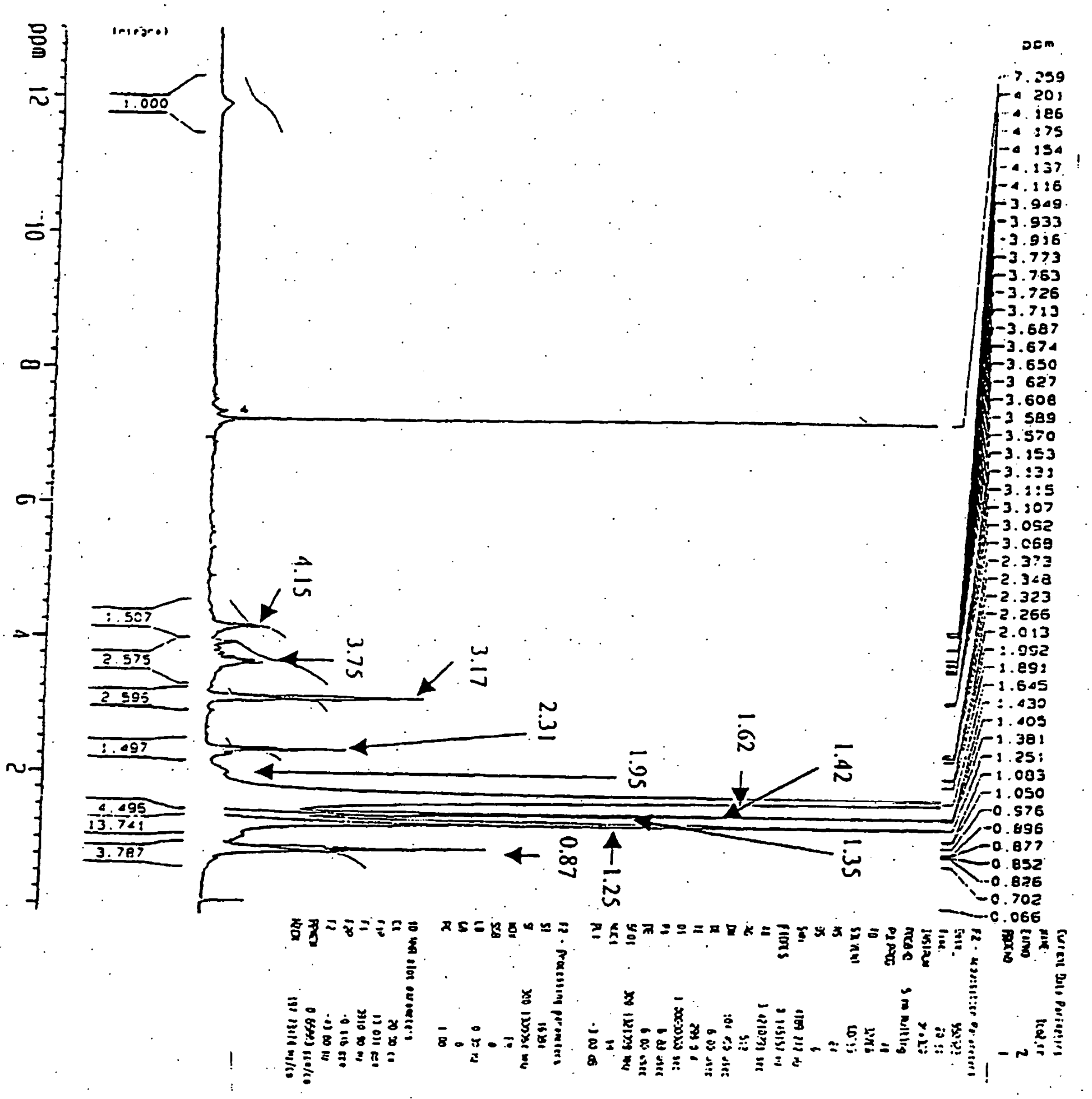

Fig 11: COSY spectrum of the purified compound.

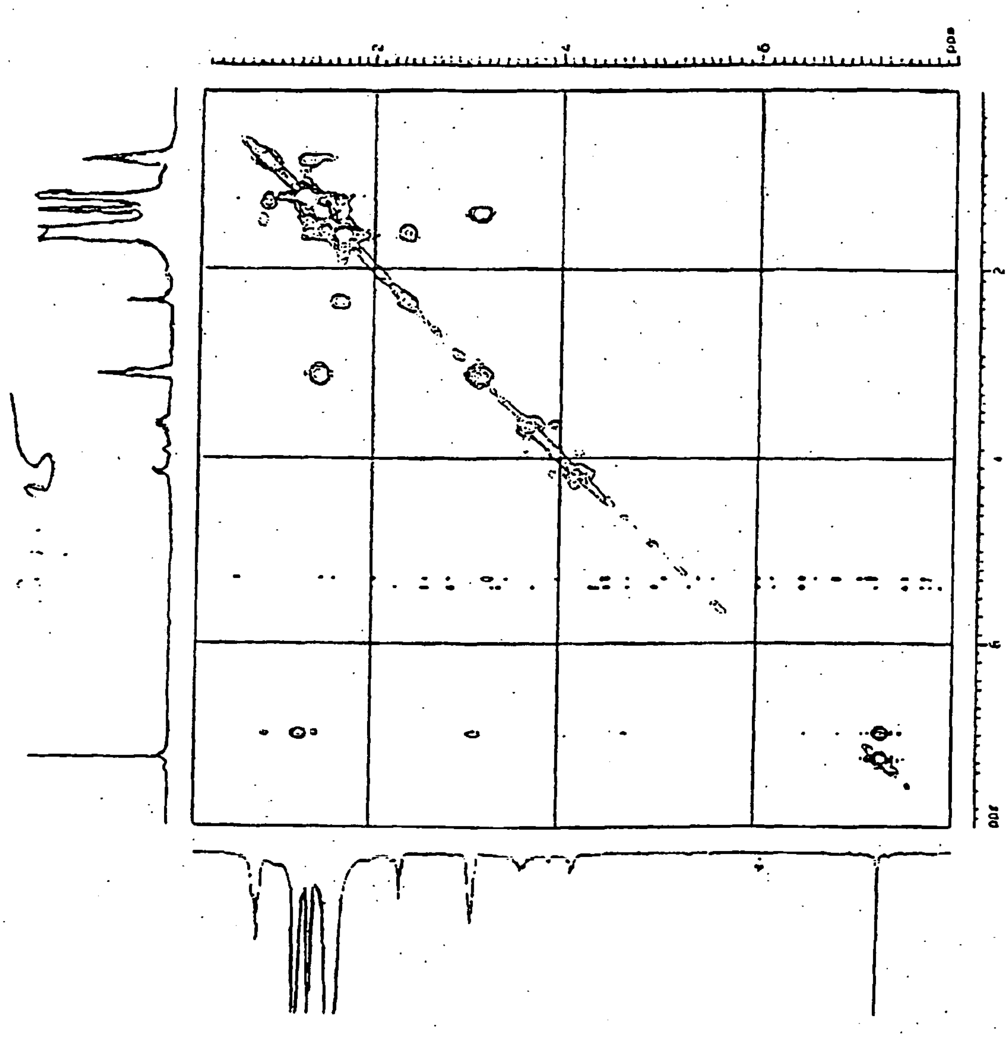

Fig 12. Percent cytotoxicity of the purified compound.

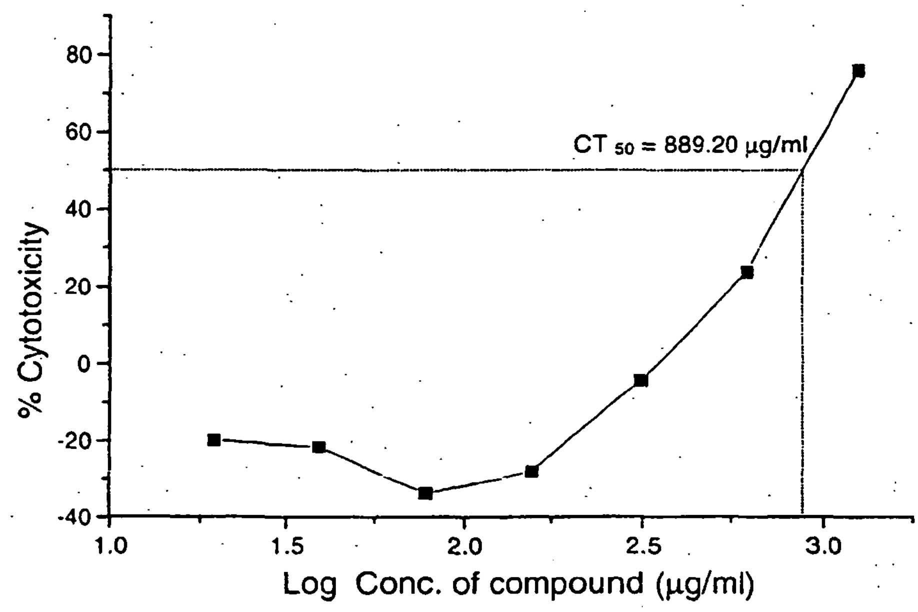

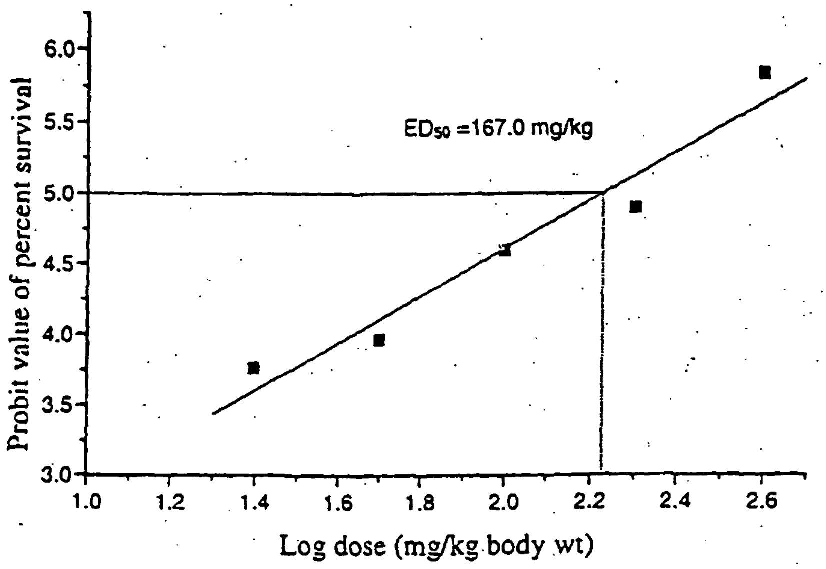


Fig 13: The dose required to protect 50% of the animals ($ED_{50}$) after treatment with compound PC-1 of *D. metel*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SARAL, R.** *Rev. Inf. Dis.,* 1991, vol. 13, 487-492
- **KOLL, B.S. ; BROWN, A.E.** *Clin. Inf. Dis.,* 1993, vol. 17, S-322-S-326
- **DENNING, D.W. ; STEVENS, D.A.** *Rev. Inf. Dis.,* 1990, vol. 12, 1147-1201
- **MEYER, J.D.** *Semin. Oncol.,* 1990, vol. 17 (6), 10-13
- **ALLENDE, M.C. ; LEE, J.W. ; FRANCIS, P. ; GARRETT, K. ; DOLLENBERG, H. ; BERENGUER, J. ; LYMAN, C. A. ; PIZZO, P.A. ; WALSH, T.J.** *Antimicrob. Agents and Chemother,* 1994, vol. 38, 518-522
- **STEVENS, D.A. ; LEE, J.Y.** *Arch. Int. Med.,* 1997, vol. 57, 1857-1862
- **GOKHALE, P.C. ; KSHIRSAGAR, N.A. ; PANDYA, S.K.** *Current Science,* 1993, vol. 65 (6), 448-454
- In Vitro susceptibility Testing of Yeast. **FORTHERGILL, A.W. ; MCGOUGH, D.A.** Clin. Microbiol. Procedure Handbook. 1995, 5.15.1-5.15.15
- Vitro susceptibility Testing of Yeasts. **FORTHERGILL A.W. ; MCGOUGH A.W.** Clin. Microbiol. Procedure Handbook. 1995, 5.15.1-5.15.15
- **DENNING, D.W. ; TUCKER, R.M. ; HANSON, L.H. ; HAMILTON, J.R. ; STEVENS, D.A.** *Arch. Int. Med.,* 1989, vol. 149, 2301-2308
- Vitro susceptibility Testing of Yeasts. **FORTHERGILL A.W. ; MCGOUGH A.W.** Clin. Microbiol. Procedure Handbook. 1995, 5.15.1-5.15.14
- **HARBOURNE J.B.** Phytochemical Methods. Chapman and Hall, 1997, 1-5
- *Indian Pharmacopoeia,* 1996, vol. 9, A101-A110
- **SUREDER P. ; JANAIAH C.** *Ind. J. Expt. Biol.,* 1987, vol. 25, 233-234
- **MOSSMAN, T.** *J. Immunol. Methods,* 1983, vol. 65, 55-63